(19) Europäisches Patentamt
European Patent Office
Office européen des brevets

(11) **EP 3 703 767 B1**

(12) **EUROPÄISCHE PATENTSCHRIFT**

(45) Veröffentlichungstag und Bekanntmachung des Hinweises auf die Patenterteilung:
**05.01.2022  Patentblatt 2022/01**

(21) Anmeldenummer: **18796436.6**

(22) Anmeldetag: **31.10.2018**

(51) Int Cl.:
*A61L 27/06* (2006.01)  *A61L 27/30* (2006.01)
*A61L 31/02* (2006.01)  *A61L 31/08* (2006.01)

(86) Internationale Anmeldenummer:
**PCT/EP2018/079896**

(87) Internationale Veröffentlichungsnummer:
**WO 2019/086551 (09.05.2019 Gazette 2019/19)**

(54) **IMPLANTAT AUS EINEM METALL**

IMPLANT MADE OF A METAL

IMPLANT EN MÉTAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.11.2017  DE 102017125635**

(43) Veröffentlichungstag der Anmeldung:
**09.09.2020  Patentblatt 2020/37**

(73) Patentinhaber: **Friedrich-Alexander-Universität Erlangen-Nürnberg**
**91054 Erlangen (DE)**

(72) Erfinder:
- **ROSIWAL, Stefan**
  **96049 Bamberg (DE)**
- **SCHUMANN, Marie**
  **79104 Freiburg (DE)**

(74) Vertreter: **Dr. Gassner & Partner mbB**
**Wetterkreuz 3**
**91058 Erlangen (DE)**

(56) Entgegenhaltungen:
EP-A1- 3 323 380    WO-A1-2014/015444
WO-A2-02/45589    DE-U1- 29 916 126

- **TSUYOSHI OCHIAI ET AL: "Application of Boron-Doped Diamond Microelectrodes for Dental Treatment with Pinpoint Ozone-Water Production", CHEMPHYSCHEM - A EUROPEAN JOURNAL OF CHEMICAL PHYSICS & PHYSICALCHEMISTRY., Bd. 14, Nr. 10, 30. November 2012 (2012-11-30), Seiten 2094-2096, XP055544525, DE ISSN: 1439-4235, DOI: 10.1002/cphc.201200845**
- **DATABASE WPI Week 201615 Thomson Scientific, London, GB; AN 2016-11166H XP002788180, -& WO 2016/017694 A1 (GC CORP) 4. Februar 2016 (2016-02-04)**
- **MARÍA ALCAIDE ET AL: "Boron-Doped Nanocrystalline Diamond Electrodes for Neural Interfaces: In vivo Biocompatibility Evaluation", FRONTIERS IN NEUROSCIENCE, Bd. 10, 8. März 2016 (2016-03-08), XP055543134, DOI: 10.3389/fnins.2016.00087**
- **DATABASE WPI Week 201763 Thomson Scientific, London, GB; AN 2017-64230T XP002788193, & JP 2017 159002 A (IMOTT KK) 14. September 2017 (2017-09-14)**
- **Katharina Neuerer: "Beeinflussung der Titankarbid-Schichtdicke bei der HFCVD-Diamantbeschichtung von Titan durch Oberflächenvorbehandlungen und Variation der Beschichtungsparameter", , 15. April 2013 (2013-04-15), XP055545200, Gefunden im Internet: URL:https://d-nb.info/1033688290/34 [gefunden am 2019-01-21] in der Anmeldung erwähnt**

- Dirk Mohn ET AL: "Electrochemical Disinfection of Dental Implants - a Proof of Concept", PLOS One, 14. Januar 2011 (2011-01-14), Seiten 1-6, XP055545318, Gefunden im Internet: URL:https://journals.plos.org/plosone/article/file?id=10.1371/journal.pone.0016157&type=printable [gefunden am 2019-01-21]
- PHILIPP SAHRMANN ET AL: "Effect of Low Direct Current on Anaerobic Multispecies Biofilm Adhering to a Titanium Implant Surface : Electrochemical Implant Disinfection", CLINICAL IMPLANT DENTISTRY AND RELATED RESEARCH, Bd. 16, Nr. 4, 21. November 2012 (2012-11-21), Seiten 552-556, XP055545321, CA ISSN: 1523-0899, DOI: 10.1111/cid.12018

## Beschreibung

Die Erfindung betrifft ein Implantat aus einem Metall. Dabei kann es sich beispielsweise um ein Zahn- oder ein Kieferimplantat oder um eine sogenannte Endoprothese, insbesondere eine Endoprothese zum Ersatz eines Knochens oder eines Gelenks, handeln. Es kann sich dabei aber auch um einen nur für eine verhältnismäßig kurze Zeit in den Körper eingeführten Gegenstand handeln.

Es ist bekannt, dass von außen eingebrachte oder endogen verschleppte Bakterien auf Implantatoberflächen adhärieren können. Dies kann zu einer Entzündungsreaktion führen, welche das umliegende Gewebe schädigt. Wegen eines Funktionsverlustes durch septische Lockerung oder der Wirkung des Implantats als Infektionsherd kann dies dazu führen, dass das Implantat operativ entfernt werden muss.

Zur Vermeidung derartiger Infektionen sind Gentamicin-Beschichtungen von Endoprothesen bekannt. Dabei wird das Antibiotikum Gentamicin aus einem Polymer-Trägermaterial lokal freigesetzt. Das Polymer-Trägermaterial wird anschließend im Körper abgebaut. Der antibakterielle Effekt wirkt jedoch nur für eine relativ kurze Zeit. Weiterhin birgt das Antibiotikum das Problem einer Resistenzbildung.

Weiterhin ist es bekannt, Endoprothesen mit antibakteriellen Kupfer- und Silberbeschichtungen zu versehen. Von diesen Beschichtungen freigesetzte Metallionen können jedoch auch eine schädigende Wirkung auf das umgebende Gewebe aufweisen.

Aus der Dissertation Neuerer, K. "Beeinflussung der Titankarbid-Schichtdicke bei der HFCVD-Diamantbeschichtung von Titan durch Oberflächenvorbehandlungen und Variation der Beschichtungsparameter" der Technischen Fakultät der Universität Erlangen-Nürnberg, 2013 ist es bekannt, Titan mittels einer heißdraht-aktivierten chemischen Gasabscheidung mit Bor-dotiertem Diamant zu beschichten. Die Beschichtung wird durch die Bor-Dotierung elektrisch leitfähig, so dass das so beschichtete Titan als Elektrode eingesetzt werden kann. Weiterhin ist es aus der Dissertation bekannt, dass sich bei der Beschichtung von Titanelektroden mit Bor-dotiertem Diamant eine Titancarbid-Zwischenschicht zwischen der Diamantschicht und dem Titansubstrat bildet.

Die Dissertation offenbart auch den Einsatz von Bor-dotierten Diamantelektroden zur elektrochemischen Wasserreinigung, insbesondere zur Desinfizierung und Aufbereitung von Trinkwasser. Der große Vorteil der Diamantelektroden ist dabei die Erzeugung von Oxidationsmitteln an der Anode aufgrund der hohen Überspannung für die Elektrolyse des Wassers. An der Elektrode werden direkt aus dem Wasser Hydroxylradikale gebildet, die zusammen mit sekundären Oxidationsmitteln, wie z. B. aktivem Chlor und Ozon, eine Reinigungswirkung erzielen. Mit elektrochemischen Zellen konnten so Legionellen, *Escherichia coli* Bakterien und andere Keime wirksam inaktiviert werden.

Aus Kraft, A., "Doped Diamond: A Compact Review on a New, Versatile Electrode Material", International Journal of Electrochemical Science, 2 (2007), Seiten 355 bis 385 ist die Verwendung von dotierten Diamantelektroden zur Wasser- und Abwasserbehandlung bekannt.

Djamel Ghernaout, "Microorganisms' Electrochemical Disinfection Phenomena". EC Microbiology 9.4 (2017), Seiten 160 bis 169 offenbart verschiedene Mechanismen der Abtötung von Mikroorganismen zur Desinfektion und verschiedene dazu geeignete Elektrodenmaterialien, wie z. B. Bor-dotierter Diamant.

Aus Garrett, D. et al., "In vivo biocompatibility of boron doped and nitrogen included conductive-diamond for use in medical implants", J Biomed Mater Res B Appl Biomater, 2016 Jan, 104(1), Seiten 19 bis 26 ist eine Untersuchung der Biokompatibilität von Implantaten aus elektrisch leitfähigem Bor-dotiertem Diamant in Meerschweinchen bekannt. Die Autoren schlussfolgern aus den Ergebnissen der Untersuchung, dass Bor-dotierter Diamant ein sicheres Material zum Implantieren ist.

In Kromka, A. et al., " Semiconducting to metallic-like boron doping of nanocrystalline diamond films and its effect on osteoblastic cells", Diamond & Related Materials 19 (2010), Seiten 190 bis 195 wird der Einfluss des Levels der Bor-Dotierung nanokristalliner Diamantfilme auf Siliziumträgern auf die Adhäsion, Proliferation und Differenzierung von osteoblastenartigen Zellen beschrieben. Alle untersuchten Substrate zeigten eine gute Biokompatibilität und stimulierten die Adhäsion und das Wachstum der untersuchten Zellen.

Booth, L. et al., "Synthesis and Characterization of Multilayered Diamond Coatings for Biomedical Implants", Materials (Basel). 2011 May; 4(5), Seiten 857 bis 868 offenbart eine mehrlagige Diamantbeschichtung mit abwechselnden nanokristallinen und mikrokristallinen Diamantschichten auf Oberflächen aus Ti-6Al-4V.

Aus Alcaide, M. et al., "Boron-Doped Nanocrystalline Diamond Electrodes for Neural Interfaces: In vivo Biocompatibility Evaluation", Frontiers in Neuroscience, Vol. 10, Artikel 87, März 2016 sind implantierbare Bor-dotierte nanokristalline Diamantelektroden zur neuronalen Stimulation bekannt.

Dirk Mohn et al: "Electrochemical Disinfection of Dental Implants - a Proof of Concept", PLOS One, 14. Januar 2011 (2011-01-14), Seiten 1-6, XP055545318, und PHILIPP SAHRMANN et al: "Effect of Low Direct Current on Anaerobic Multispecies Biofilm Adhering to a Titanium Implant Surface: Electrochemical Implant Disinfection", CLINICAL IMPLANT DENTISTRY AND RELATED RESEARCH, Bd. 16, Nr. 4, 21. November 2012 (2012-11-21), Seiten 552-556, XP055545321, CA, ISSN: 1523-0899, beschreiben als Elektrode geschaltete Titan-Implantate, mit elektrolytisch erzeugten, oxidierenden und desinfizierenden Produkten wie Chlor, Hypochlorit-Anionen, Chlordioxid, Hydroxid-Radikalen und reaktivem Sauerstoff.

Aufgabe ist es, ein Metallimplantat mit einer Oberflächenbeschichtung mit Bor-dotiertem Diamant zur Verwendung bei einer alternativen therapeutischen Behandlung anzugeben.

Die Aufgabe wird durch die Merkmale des Patentanspruchs 1 gelöst.

Die Erfindung wird durch die Ansprüche definiert. Sie betrifft an einem Implantat aus einem Metall oder mit einer Oberfläche aus einem Metall erzeugte oxidierende Produkte mit antimikrobieller Wirkung zur Verwendung bei einer therapeutischen Behandlung, wobei das Implantat auf dessen/der Oberfläche eine Beschichtung mit polykristallinem dotiertem elektrisch leitfähigem Diamant aufweist, wobei die therapeutische Behandlung eine Therapie einer mikrobiellen Infektion durch Bakterien am Implantat eines menschlichen oder tierischen Körpers ist, wobei die therapeutische Behandlung die Erzeugung der Produkte mit antimikrobieller Wirkung direkt am Implantat umfasst, wobei die Produkte mit antimikrobieller Wirkung $C_2O_6^{2-}$, $S_2O_8^{2-}$ und/oder $P_2O_8^{4-}$-Ionen sind, wobei das Implantat in einem elektrochemischen System in dem Körper als Anode geschaltet ist, wobei das elektrochemische System neben der Anode eine Kathode, eine jeweils mit der Anode und der Kathode elektrisch leitend verbundene Stromquelle und einen eine Körperflüssigkeit umfassenden oder aus einer Körperflüssigkeit bestehenden Elektrolyten umfasst oder aus der Anode, einer Kathode, einer jeweils mit der Anode und der Kathode elektrisch leitend verbundenen Stromquelle und einem eine Körperflüssigkeit umfassenden oder aus einer Körperflüssigkeit bestehenden Elektrolyten besteht, oder wobei das Implantat innerhalb eines elektrischen Feldes angeordnet ist, wodurch durch Induktion auf dem Implantat an einer ersten Stelle eine negative Ladung und an einer zweiten Stelle eine positive Ladung induziert wird, wodurch die erste Stelle in einem elektrochemischen System zur Anode und die zweite Stelle in dem elektrochemischen System zur Kathode wird, wobei das elektrochemische System neben dem Implantat einen eine Körperflüssigkeit umfassenden oder aus einer Körperflüssigkeit bestehenden Elektrolyten umfasst oder aus dem Implantat und einem eine Körperflüssigkeit umfassenden oder aus einer Körperflüssigkeit bestehenden Elektrolyten besteht. Zweckmä-ßige Ausgestaltungen ergeben sich aus den Merkmalen der Patentansprüche 2 bis 15. Gegenstände die nicht unter den Umfang der Ansprüche fallen dienen lediglich der Information.

Beschrieben ist ein Implantat aus einem Metall oder mit einer Oberfläche aus einem Metall zur Verwendung bei einer therapeutischen Behandlung, wobei das Implantat auf dessen/der Oberfläche eine Beschichtung mit polykristallinem dotiertem elektrisch leitfähigem Diamant aufweist. Die therapeutische Behandlung ist dabei eine Therapie einer, insbesondere mit einer Entzündungsreaktion einhergehenden, mikrobiellen Infektion eines menschlichen oder tierischen Körpers. Die Therapie kann dabei eine der mikrobiellen Infektion vorbeugende Therapie oder eine die mikrobielle Infektion heilende Therapie sein.

Dabei ist das Implantat in einem elektrochemischen System in dem Körper als Anode geschaltet. Das elektrochemische System umfasst dabei neben der Anode eine Kathode, eine jeweils mit der Anode und der Kathode elektrisch leitend verbundene Stromquelle und einen eine Körperflüssigkeit umfassenden oder aus einer Körperflüssigkeit bestehenden Elektrolyten oder es besteht dabei aus der Anode, einer Kathode, einer jeweils mit der Anode und der Kathode elektrisch leitend verbundenen Stromquelle und einem eine Körperflüssigkeit umfassenden oder aus einer Körperflüssigkeit bestehenden Elektrolyten. Die Kathode kann dabei aus einem üblichen Elektrodenmaterial bestehen. Das Elektrodenmaterial kann beispielsweise Platin, Stahl, insbesondere Edelstahl, Kohlenstoff, insbesondere in Form von Graphen, oder, insbesondere bei einer in dem menschlichen oder tierischen Körper angeordneten Kathode, Titan oder mit Bor-dotiertem Diamant beschichtetes Titan oder Stahl sein. Die elektrisch leitende Verbindung der Anode und der Kathode mit der Stromquelle kann über einen elektrisch leitenden Draht oder Metallstift erfolgen. Die Stromquelle ist im Allgemeinen eine Gleichstromquelle, die beispielsweise in Form einer Batterie oder eines Akkus in dem menschlichen oder tierischen Körper angeordnet sein kann. Sie kann aber auch außerhalb des menschlichen oder tierischen Körpers angeordnet sein und beispielsweise eine Batterie oder ein Akku oder ein eine definierte Gleichstromspannung bereitstellender Transformator oder bereitstellendes Netzteil sein.

Alternativ ist es möglich, dass das Implantat innerhalb eines elektrischen Feldes angeordnet ist, wodurch durch Induktion auf dem Implantat an einer ersten Stelle eine negative Ladung und an einer zweiten Stelle eine positive Ladung induziert wird, wodurch die erste Stelle in einem elektrochemischen System zur Anode und die zweite Stelle in dem elektrochemischen System zur Kathode wird, wobei das elektrochemische System neben dem Implantat einen eine Körperflüssigkeit umfassenden oder aus einer Körperflüssigkeit bestehenden Elektrolyten umfasst oder aus dem Implantat und einem eine Körperflüssigkeit umfassenden oder aus einer Körperflüssigkeit bestehenden Elektrolyten besteht. Eine solche Anordnung wird als bipolare Anordnung bezeichnet.

Das elektrische Feld kann dabei zwischen einer weiteren Anode und einer weiteren Kathode innerhalb des elektrochemischen Systems aufgebaut sein. Das elektrochemische System umfasst in diesem Fall zusätzlich neben der weiteren Anode und der weiteren Kathode eine mit der weiteren Anode und der weiteren Kathode elektrisch leitend verbundene Stromquelle, oder es besteht zusätzlich aus der weiteren Anode und der weiteren Kathode sowie einer mit der weiteren Anode und der weiteren Kathode elektrisch leitend verbundenen Stromquelle.

Der Elektrolyt in dem elektrochemischen System kann eine elektrisch leitende Hilfsflüssigkeit umfassen oder aus der Körperflüssigkeit und der elektrisch leitenden Hilfsflüssigkeit bestehen. Die elektrisch leitende Hilfsflüssigkeit ist dabei jeweils eine einen elektrischen Kontakt der weiteren Anode und der weiteren Kathode mit der Körperflüssigkeit herstellende Flüssigkeit. Die Hilfsflüssigkeit enthält daher Ionen und kann beispielsweise eine Kochsalzlösung sein. Die Hilfsflüssigkeit kann in Form eines Gels vorliegen. Wenn die weitere Anode und die weitere Kathode auf der Haut des menschlichen oder tierischen Körpers angeordnet sind, kann die Hilfsflüssigkeit jeweils zwischen der Haut und der weiteren Anode und der Haut und der weiteren Kathode angebracht sein, um den elektrischen Kontakt der weiteren Anode und der weiteren Kathode mit der Körperflüssigkeit herzustellen.

Das elektrische Feld kann auch zwischen mindestens zwei, insbesondere außerhalb des elektrochemischen Systems angeordneten, Platten eines Kondensators oder durch mindestens eine, insbesondere außerhalb des elektrochemischen Systems angeordnete, elektrische Spule aufgebaut sein. Die beiden Platten des Kondensators bilden dabei ein Kondensatorplattenpaar.

Weiterhin ist es möglich, dass die Ausrichtung des elektrischen Feldes während der therapeutischen Behandlung verändert wird. Das kann beispielsweise dadurch erfolgen, dass die mindestens zwei Platten des Kondensators um den Körper oder einen Teils des Körpers, in welchem sich das Implantat befindet, rotiert werden. Dadurch rotieren dann auch das elektrische Feld und die Positionen der ersten und der zweiten Stelle auf dem Implantat. Bei der therapeutischen Behandlung ist es auch möglich, die Platten des Kondensators umzupolen, so dass die Position der ersten und der zweiten Stelle auf dem Implantat wechseln. Es ist auch möglich, mehr als ein Kondensatorplattenpaar vorzusehen und das elektrische Feld sequentiell oder alternierend zwischen je einem der Kondensatorplattenpaare aufzubauen. Auch dadurch ist es möglich, die Ausrichtung des elektrischen Feldes während der therapeutischen Behandlung zu verändern und insbesondere das elektrische Feld zu rotieren.

Bei dem tierischen Körper kann es sich insbesondere um einen tierischen Körper eines Säugetiers handeln. Die Körperflüssigkeit kann auch eine Körperflüssigkeit sein, welche in einem Körpergewebe enthalten ist. Bei der Körperflüssigkeit kann es sich beispielsweise auch um Blut, Lymphe oder Schweiß oder mehrere unterschiedliche Flüssigkeiten im menschlichen oder tierischen Körper oder eine Mischung von mindestens zwei dieser Flüssigkeiten handeln. Bei dem Körpergewebe kann es sich um ein Hartgewebe oder ein Weichgewebe handeln.

Die elektrische Leitfähigkeit des polykristallinen Diamanten wird durch die Dotierung erreicht. Der Diamant kann dazu mit Bor oder Phosphor dotiert sein.

Die Erfinder der vorliegenden Erfindung haben erkannt, dass ein Implantat aus einem Metall oder mit einer Oberfläche aus Metall mit einer Oberflächenbeschichtung mit polykristallinem dotiertem elektrisch leitfähigem Diamant sehr gut zur Therapie einer mikrobiellen Infektion eines menschlichen oder tierischen Körpers, insbesondere aufgrund einer mikrobiellen Besiedlung des Implantats, verwendet werden kann. Die Erfinder haben festgestellt, dass sich ein solches Implantat sowohl zur Inaktivierung einzelner adhärenter Zellen als auch zur Inaktivierung von in komplexen Biofilmen enthaltenen Mikroorganismen eignet. Dazu ist eine Spannung im Bereich von 3 V bis 15 V, insbesondere 4 V bis 6 V gut geeignet. Die genannte Inaktivierung kann aber auch schon mit einer Spannung von mindestens 2 V erreicht werden. Nach der Inaktivierung von in den Biofilmen enthaltenen Mikroorganismen werden die Biofilme und die Mikroorganismen üblicherweise durch körpereigene Mechanismen abgebaut.

Bor-dotierter Diamant (BDD) weist ein Potentialfenster von 3,5 V auf. Mit in einem elektrochemischen System als Anode geschaltetem BDD ist es innerhalb dieses Potentialfensters möglich, direkt an der Anode OH-Radikale zu erzeugen, weil innerhalb dieses Potentialfensters eine elektrolytische Abspaltung von Sauerstoff aus Wasser an der Anode unterbleibt. An der Anode erfolgt dabei die folgende Reaktion:

$$BDD + H_2O \rightarrow BDD\ (OH\bullet) + H^+ + e^-$$

Kathodisch bildet sich in diesem Spannungsbereich Wasserstoff gemäß der folgenden Reaktion:

$$2H_2O + 2e^- \rightarrow 2H_2 + 2OH^-$$

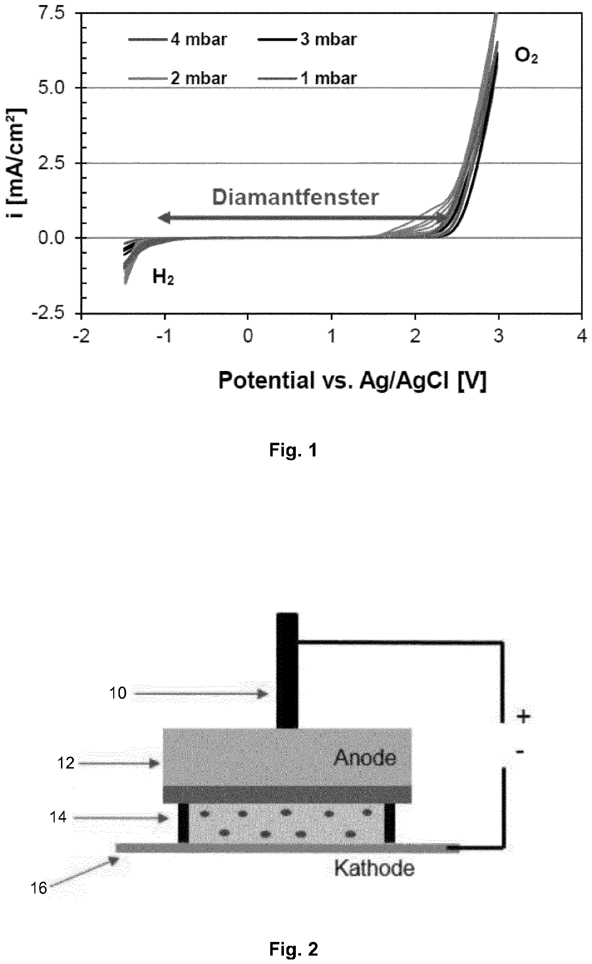

Im elektrochemischen Fenster können neben OH-Radikalen weitere oxidierende Produkte, wie z. B. $O_3$, $O_2$, $Cl_2$, $H_2O_2$, $C_2O_6^{2-}$, $S_2O_8^{2-}$, $P_2O_8^{4-}$ oder $ClO^{2-}$, gebildet werden. Sobald das angelegte Potential größer oder kleiner als das Potentialfenster ist, wird Wasser zersetzt. Fig. 1 verdeutlicht diesen Zusammenhang.

Die Stromdichte-Potentialkurve gemäß Fig. 1 wurde an Bor-dotierten Diamantschichten mit Schichtdicken im Bereich von 3 bis 8 $\mu$m auf p-dotierten Si-Wafern gegen eine Ag/AgCl-Elektrode gemessen. Die Diamantschichten wurden bei verschiedenen Drücken hergestellt. Als Elektrolyt wurde dabei Essigsäure mit $Na_2SO_4$ als Leitsalz verwendet. Innerhalb des Diamantfensters bilden sich aufgrund der Überspannung von Diamant oxidierende Produkte. Außerhalb dessen wird zusätzlich Wasser gespalten. Der Anstieg bei circa 2,8 V zeigt den Beginn der anodischen Sauerstoffentwicklung durch die Zersetzung des Wassers. Bei circa -1,3 V fällt die Kurve aufgrund der kathodischen Wasserstoffproduktion. Die Kurve ist nicht allgemein gültig. Die Größe des Diamantfensters kann bei einer Stromdichte-Potential-Messung mit einer anderen Kathode und/oder anderen Anode davon abweichen. So kann eine Anode mit einer Diamantbeschichtung mit einer abweichenden Korngröße innerhalb des Diamanten eine andere Stromdichte-Potentialkurve als die in Fig. 1 dargestellte ergeben. Beispielsweise ergibt eine Anode mit einer überwiegend aus Diamant mit einer Korngröße im Nanobereich bestehenden Diamantschicht ein kleineres Potentialfenster als das aus Fig. 1 ersichtliche Potentialfenster. Die Diamantschicht der bei dem in Fig. 1 dargestellten Versuch verwendeten Anode wies eine Korngröße im Bereich von 2 bis 3 $\mu$m auf.

Die Erfinder haben herausgefunden, dass sich Mikroorganismen, insbesondere Bakterien, welche auf der Oberfläche aus Bor-dotiertem Diamant sitzen, gut elektrochemisch so schädigen lassen, dass diese absterben, wenn das Implantat aus Metall mit der Diamantoberfläche als Anode geschaltet ist. Dabei reicht das Anliegen einer minimalen Spannung, beispielsweise von 1,23 V, bereits dazu aus, Produkte mit antimikrobieller Wirkung an der Anode entstehen zu lassen. Die antimikrobielle Wirkung des Implantats kann dabei einerseits auf der Entstehung von Hydroxylradikalen (OH•) und andererseits auf der Entstehung von Oxidationsmitteln oder Reaktionsprodukten der Hydroxylradikale beruhen. Oxidationsmittel, d. h. oxidierende Produkte, können bereits bei geringeren Potentialen als dem für die Entstehung von Hydroxylradikalen erforderlichen Potential entstehen. Die folgende Tabelle 1 zeigt, welche oxidierenden Produkte bei welchem Potential innerhalb des Diamantfensters entstehen können.

Tabelle 1:

| Oxidierende Produkte | BDD-Elektrodenpotential [V] |
|---|---|
| $O_2$ | 1,23 |
| $Cl_2$ | 1,36 |
| $ClO^{2-}$ | 1,57 |
| $H_2O_2$ | 1,77 |
| $C_2O_6^{2-}$ | 1,80 |
| $S_2O_8^{2-}$ | 2,01 |
| $P_2O_8^{4-}$ | 2,07 |
| $O_3$ | 2,07 |
| **OH•** | **2,80** |

Die therapeutische Behandlung bei der das Implantat als Anode in dem elektrochemischen System geschaltet ist, d. h. bei der mittels der Stromquelle eine elektrische Spannung an das elektrochemische System angelegt wird, erfolgt üblicherweise jeweils nur für wenige Minuten. Sie kann aber mehrfach wiederholt werden. Die dazu angelegte Spannung kann auch außerhalb des Diamantfensters liegen. Dabei können auch gasförmige Produkte, wie beispielsweise $O_2$, $O_3$ oder aus dem Kohlenstoff der das Implantat umgebenden tierischen oder menschlichen Zellen $CO_2$ oder CO entstehen. Bei Implantaten innerhalb eines geschlossenen Bereichs des Körpers sollte die Spannung dabei so gewählt werden, dass die Menge der an der Anode entstehenden gasförmigen Produkte so gering ist, dass sich diese unmittelbar nach deren Entstehen in der Körperflüssigkeit lösen können. Die Anode wird in der insgesamt relativ kurzen Behandlungszeit und bei der geringen Spannung nicht erkennbar angegriffen.

Durchgeführte Experimente in einem Zellkulturmodell haben gezeigt, dass es bei einer nur für wenige Minuten am Stück erfolgenden Behandlung, die damit einer möglichen tatsächlichen therapeutischen Behandlung entspricht, zu keiner oder zumindest zu keiner wesentlichen Schädigung von das Implantat im menschlichen oder tierischen Körper umgebenden gesunden Zellen und damit auch nicht zu einer wesentlichen Schädigung von das Implantat im menschlichen oder tierischen Körper umgebendem gesundem Gewebe kommt. Eine Schädigung inflammatorischer oder durch eine Entzündung vorgeschädigter körpereigener Zellen ist dagegen möglich und bei der Therapie einer mikrobiellen Infektion durchaus erwünscht. Mit der allenfalls geringen Schädlichkeit gegenüber den das Implantat im menschlichen oder tierischen Körper umgebenden gesunden Zellen unterscheidet sich das hier beschriebene Implantat von einem Silberionen abgebenden silberbeschichteten oder einem sonstigen toxische Metallionen abgebenden Implantat, welches toxisch auf solche Zellen wirken kann. Da die toxischen Ionen permanent von einem solchen Implantat abgegeben werden, von dem hier beschriebenen Implantat jedoch nur bei Anlegen einer Spannung eine antimikrobielle Wirkung ausgeht, kann auch ein ggf. bisher unerkannter das Gewebe schädigender Effekt nur während der kurzen Zeit auftreten, in welcher eine Spannung angelegt ist. Die antimikrobielle Wirkung des Implantats kann jederzeit an- oder abgeschaltet werden.

Das Implantat ermöglicht die Bekämpfung einer Implantat-assoziierten Infektion und die Vermeidung einer durch eine solche Infektion verursachten Komplikation. Bei der Komplikation kann es sich z. B. um eine periimplantäre Mukositis bei einem Zahnimplantat, einen Funktionsverlust des Implantats durch eine septische Lockerung des Implantats, eine Wirkung des Implantats als Infektionsherd oder eine Zerstörung von das Implantat umgebendem Gewebe durch eine Entzündungsreaktion handeln.

Bei der mikrobiellen Infektion handelt es sich um eine bakterielle Infektion.

Das Metall kann Titan oder eine Titan enthaltende Legierung sein. Titan ist durch seine Härte und seine gute Einheilbarkeit als Implantat, insbesondere beim Ersatz von Gelenken oder beim Einsatz in einen Kieferknochen, als Material für ein Implantat besonders gut geeignet. Die Beschichtung von Titan mit dotiertem Diamant ist problemlos mittels bekannter Technik möglich.

Bei der Legierung kann es sich um Ti-6Al-4V, Ti-6Al-7Nb oder eine sonstige Legierung handeln, die neben Titan Aluminium und/oder Niob und/oder Eisen und/oder Molybdän enthält.

Die Beschichtung kann eine Beschichtung sein, die durch heißdraht-aktivierte chemische Gasphasenabscheidung (hot filament chemical vapor deposition = HFCVD) oder durch mikrowellenplasmaunterstützte chemische Gasphasenabscheidung (microwave plasma-assisted chemical vapor deposition = MPCVD) oder durch eine sonstige chemische Gasphasenabscheidung (chemical vapor deposition = CVD) an der Oberfläche des Implantats in einer eine borhaltige Verbindung enthaltenden Gasphase erzeugt ist. Bei der borhaltigen Verbindung kann es sich beispielsweise um Bortrioxid, Diboran, Triethylboran oder Trimethylborat handeln. Die Beschichtung von Titansubstraten mit Diamant für den Einsatz als Elektrodenmaterial ist im Stand der Technik bekannt. Im industriellen Dauerbetrieb weisen diese Elektroden zwar keine ausreichende Stabilität über einen Betriebszeitraum von etwa 100 Stunden auf, beim erfindungsgemäß vorgesehenen Einsatzzweck, bei welchem das Implantat nur für eine begrenzte und insgesamt relativ kurze Zeit von üblicherweise höchstens 10 Stunden als Anode geschaltet ist, kommt dies jedoch nicht zum Tragen.

Wenn das Metall Titan oder die Titan enthaltende Legierung ist, kann sich zwischen dem Titan oder der Legierung und der Beschichtung eine Zwischenschicht aus Titancarbid (TiC) befinden. Titancarbid bildet sich während des Beschichtungsprozesses durch Reaktion von Titan mit der kohlenstoffhaltigen Atmosphäre spontan auf der Titanoberfläche oder der Titan enthaltenden Oberfläche. Bei der Beschichtung mittels chemischer Gasphasenabscheidung konkurrieren das Diamantwachstum und das Titancarbidwachstum um den in der Gasphase vorhandenen Kohlenstoff. Sobald sich jedoch eine geschlossene Diamantschicht ausgebildet hat, ist das Wachstum der Titancarbidschicht beendet. Die Titancarbidschicht vermittelt eine gute Haftung der Diamantschicht auf dem Titan. Sie beeinflusst jedoch auch wesentlich die Haltbarkeit der gebildeten Diamantelektrode. Durch eine hohe Temperatur des Titansubstrats im Beschichtungsprozess wird die Bildung einer dicken und spröden Titancarbidschicht gefördert, deren hohe thermische Spannung ein Abplatzen der Diamantschicht und somit eine Verringerung der Stabilität der Elektroden bewirken kann.

Um die Dicke der Titancarbidschicht zu reduzieren und damit die elektrochemische Beständigkeit der Diamantelektroden zu verbessern, können beim Beschichten eine verhältnismäßig niedrige Substrattemperatur und ein verhältnismäßig hoher Methanfluss gewählt werden. Eine weitere Möglichkeit der Verringerung der Dicke der Titancarbidschicht besteht in einer Oxidierung der Titanoberfläche vor der Beschichtung.

Das Implantat hat sich als besonders gut antimikrobiell wirkend erwiesen, wenn die Zwischenschicht eine Schichtdicke von 3 $\mu$m, insbesondere 2,5 $\mu$m, insbesondere 2 $\mu$m, nicht überschreitet. Für eine gute Beständigkeit der Oberflächenbeschichtung hat sich außerdem eine Schichtdicke der Zwischenschicht von mindestens 100 nm als günstig erwiesen. Die Diamantschicht kann eine Schichtdicke von 100 nm bis 10 $\mu$m, insbesondere 2 $\mu$m bis 3 $\mu$m, insbesondere 2 $\mu$m bis 2,8 $\mu$m, aufweisen.

Das Implantat kann ein von außerhalb des Körpers ohne chirurgischen Eingriff zugängliches Zahn- oder Kieferimplantat sein und für die Therapie von außerhalb des Körpers elektrisch kontaktiert werden. Die Kathode kann dazu in der Mundhöhle oder auf der Haut so platziert werden, dass ein Stromfluss möglich ist. Die Stromquelle befindet sich dabei üblicherweise außerhalb des Körpers.

Es ist auch möglich, dass das Implantat in ein Inneres des Körpers eingesetzt ist und ein elektrischer Leiter, insbesondere ein elektrisch leitender Draht, vom Implantat auf eine Außenseite des Körpers geführt ist und für die Therapie auf der Außenseite elektrisch kontaktiert wird. Das Implantat kann auch ein Kurzzeitimplantat sein. Bei einem Kurzzeitimplantat kann es sich beispielsweise um einen für kurze Zeit im Körper verlegten Spezialdraht, etwa bei einer Herzkatheteruntersuchung oder eine für eine kurze Zeit im Körper verlegte Elektrode handeln.

Bei einer anderen Ausgestaltung ist der elektrische Leiter vom Implantat zu einer Kontaktstelle unter der Haut geführt, die für die Therapie von außen durch eine Punktion elektrisch kontaktiert wird. In beiden Fällen kann die Kathode durch eine Punktion in den Körper eingeführt oder auf der Haut platziert werden.

Bei einer weiteren alternativen Ausgestaltung ist der elektrische Leiter vom Implantat über einen elektrischen Gleichrichter zu einer, insbesondere nicht mehr als 3 cm unter der Haut angeordneten, Spule geführt und mit dieser elektrisch leitend verbunden. Die Spule ist in diesem Fall weiterhin über den elektrischen Gleichrichter elektrisch leitend mit der Kathode verbunden. Die Kathode ist dabei ebenfalls im Körper angeordnet. Für die Therapie wird in der Spule von außerhalb des Körpers durch Induktion ein Stromfluss induziert, durch welchen die Kathode und die Anode unter Strom gesetzt werden. Dies ermöglicht eine Therapie ohne eine Punktion und ohne einen, außer dem zum Einsetzen des Implantats erforderlichen chirurgischen Eingriff, weiteren chirurgischen Eingriff in den Körper. Die Spule bildet dabei die Stromquelle oder zumindest einen Teil der Stromquelle, der von einer externen Stromquelle Energie mittels Induktion zugeführt wird.

Nachfolgend wird die Erfindung anhand von Ausführungsbeispielen näher erläutert. Es zeigen:

Fig. 1        eine Stromdichte-Potentialkurve an Bor-dotierten Diamantschichten,

Fig. 2        einen schematisch im Querschnitt dargestellten Versuchsaufbau zur Inaktivierung von *E. coli* Bakterien in einem Agar-Biofilm,

Fig. 3.       einen schematisch im Querschnitt dargestellten alternativen Versuchsaufbau zur Inaktivierung von *E. coli* Bakterien in einem Agar-Biofilm,

Fig. 4.       einen schematisch im Querschnitt dargestellten Versuchsaufbau zur Inaktivierung von initial direkt an einer BDD-Anode adhärierten *S. gordonii* Bakterien,

Fig. 5        ein Diagramm zur Darstellung der Abhängigkeit der Anzahl der *E. coli* Kolonien von der Raumladungsdichte bei konstanter Spannung von 4,2 V,

Fig. 6        ein Diagramm zur Darstellung der Anzahl der *E. coli* Kolonien in Abhängigkeit von der Raumladungsdichte und dem Versuchsaufbau,

Fig. 7        ein Diagramm zur Darstellung der von *S. gordonii* auf BDD-beschichteten Titanplättchen besiedelten Fläche in Abhängigkeit von der Flächenladungsdichte und der angelegten Spannung,

Fig. 8        einen schematisch im Querschnitt dargestellten Versuchsaufbau zur Inaktivierung von *Staphylococcus epidermidis* und *Bacillus subtilis* in einem aufgebohrten Wurzelkanal eines menschlichen Zahns,

Fig. 9        ein Diagramm zur Darstellung der Abhängigkeit des Wachstums von *Staphylococcus epidermidis* von der Ladungsmenge und der Dauer der Behandlung des Wurzelkanals und

Fig. 10       ein Diagramm zur Darstellung der Abhängigkeit des Wachstums von *Bacillus subtilis* von der Ladungsmenge und der Dauer der Behandlung des Wurzelkanals.

**Experimentelles**

**CVD-Diamantbeschichtung von Titanplättchen**

Als Substratwerkstoff wurde Reintitan nach ASTM-Norm F-67 Grade 4 und gemäß ISO-Norm 5832-2 der Fa. L.Klein SA verwendet. Die chemische Zusammensetzung ist in der nachfolgenden Tabelle 2 aufgeführt.

Tabelle 2: Chemische Zusammensetzung von Titan Grade 4 nach ASTM-Norm F-67.

| C | N | O | Fe | H | Ti |
|---|---|---|---|---|---|
| 0,0050% | 0,0035% | 0,2650 | 0,0200% | 0,0032% | Rest |

Die daraus mittels Accutom (Fa.Struers) getrennten Probenscheiben weisen einen Durchmesser von 12 mm und eine Höhe von 1,8 mm auf.

Es wurde eine standardmäßige Probenvorbehandlung durchgeführt, die aus den Schritten Feinstrahlen, Ätzen und Bekeimen besteht. Das Feinstrahlen fand händisch mit Siliziumcarbid (SiC)-Partikeln an einer Anlage der Fa. Wassermann statt. Zur Einstellung unterschiedlicher Rauheiten wurden feinere F320 (17 bis 74 $\mu$m) SiC-Partikel für eine glattere oder gröbere F80 (125 bis 300 $\mu$m) SiC-Partikel für eine rauere Titanoberfläche verwendet. Der Strahldruck lag bei ca. 2,5 bar. Anschließend wurde zwischen 80 °C und 90 °C in wässriger Lösung aus 10% $H_2SO_4$ und 10% HCl für 10 min geätzt. Dadurch erhöht sich die Mikrorauheit der Substratoberfläche. Im letzten Schritt wurden die Proben mit einer Verdünnung der 5 Gew.-%igen wässrigen Nanodiamantpartikellösung der Fa. Carbodeon 5 min in einem Ultraschallbad bekeimt. Somit erhöht sich die Keimdichte auf der Substratoberfläche als Vorraussetzung für eine geschlossene Diamantschicht. Hierfür kam die Diamantsuspension "ANDANTE" der Firma Carbodeon Ltd. Oy, Pakkalankuja 5, 01510 Vantaa, Finnland im Verhältnis 1:1000 mit Ethanol zum Einsatz.

Die Beschichtungs- und Filamenteinfahrprozesse wurden mit der Hot Filament CVD (HFCVD) Anlage der Fa. CemeCon vom Typ CC800 Dia-8 durchgeführt. Die Filamenthalter aus Kupfer wurden in beiden Prozessen in Schiene 1 und 2 angeordnet und waren jeweils mit 42 Wolframdrähten mit einer Länge von 220 mm bespannt. Als Filamentmaterial diente AKS-dotierter Wolframdraht mit 0,11 mm Durchmesser.

Die Einfahrprozesse fanden bei einem Startdruck von 6500 mPa, 6 mbar Prozessdruck, einem Methanfluss von 16 min und 1000 min Wasserstofffluss über einen Zeitraum von 18 h statt. Mithilfe eines Zweikanal-Pyrometers, welches vor dem Sichtfenster der geschlossenen HFCVD-Anlage angeordnet war, wurde die Filamenttemperatur kontrolliert.

Es wurden 50 Titanplättchen mit Mikro-Diamant und 30 Titanplättchen mit Nano-Diamant beschichtet. Bei allen HFCVD-Prozessen betrug der Startdruck 6000 mPa und der Prozessdruck 6 mbar. Die Probentemperatur von 820 °C und die Filamenttemperatur von 2160 °C wurden eingeregelt. Jeder Beschichtungsprozess besteht aus drei Segmenten mit unterschiedlichen Gasflüssen. Der Unterschied zwischen Mikro- und Nanobeschichtungsprozess liegt im Methangehalt, der bei einer Nanobeschichtung höher ist. Die nachfolgende Tabelle 3 zeigt die Prozessparameter der Diamantbeschichtungen.

Tabelle 3: Prozessparameter und Eigenschaften der Mikro- und Nanodiamant-Beschichtungen.

| Prozessnummer | Prozessdauer [h] | $H_2$-Fluss [mln] | $CH_4$-Gehalt [%] | TMB-Gehalt [mln] | Stromstärke [A] | Schichtdicke [$\mu m$] |
|---|---|---|---|---|---|---|
| K518 Mikro F80/F320 | 1,5/ 10,5/ 0,02/ | 3000/ 2000/ 1000 | 1,6 | 0,27/ 0,18/ 0,00 | 80/ 82/ 15 | 2,5 (F80)/ 1,5 (F320) |
| K561 Mikro F320 | 1,5/ 12,5/ 0,02 | 3000/ 2000/ 1000 | 1,6 | 0,27/ 0,18/ 0,00 | 60/ 86/ 15 | 8,2 |
| K571 Mikro F320 | 1,5/ 18,5/ 0,02 | 3000/ 2000/ 1000 | 1,6 | 0,27/ 0,18/ 0,00 | 60/ 80/ 15 | 6,1 |
| K553 Nano F320 | 1,5/ 12,5/ 0,02 | 3000/ 2000/ 1000 | 3,0/ 3,5/ 1,6 | 0,27/ 0,18/ 0,00 | 80/ 84/ 15 | 0,8 |
| K575 Nano F320 | 1,5/ 18,5/ 0,02 | 3000/ 2000/ 1000 | 3,0/ 3,5/ 1,6 | 0,27/ 0,18/ 0,00 | 80/ 88/ 15 | 3,7 |

**Inaktivierung von *Escherichia coli***

**Herstellung eines *E. coli*-Agar-Biofilms**

Zur Erzeugung künstlicher Biofilme erfolgte eine Zugabe von nicht pathogenen *Escherichia coli*-K12 498 (*E. coli*) der Deutschen Sammlung von Mikroorganismen und Zellkulturen GmbH (DSMZ) aus einer Vorkultur in flüssiges Standard 1 Nährmedium (St. 1). St. 1 mit Agar-Agar wird nachfolgend als St. 1 Agar bezeichnet. Die Bestandteile dieses St. 1 Agars sind in nachfolgender Tabelle 4 aufgelistet.

Tabelle 4: Inhaltsstoffe des Standard 1 Agar-Nährmediums in 400 ml VE-Wasser.

| Inhaltsstoffe | Menge [g] | Hersteller/ Artikelnummer |
|---|---|---|
| Glukose-Monohydrat | 0,44 | Carl Roth GmbH / 6780.1 |
| Natriumchlorid | 2,34 | Carl Roth GmbH / 3957.1 |
| Agar-Agar | 8,00 | Carl Roth GmbH / 5210.3 |
| Hefeextrakt | 1,20 | Carl Roth GmbH / 2363.3 |
| Pepton aus Casein | 6,00 | Merck KGaA / 1.02239.0500 |

Der pH-Wert wurde mittels Zugabe von Natriumhydroxid auf 7,4 eingestellt. In Petrischalen ausplattiert erstarrte der St. 1 Agar nach wenigen Minuten und diente als Nährboden. 10 ml St. 1 Agar wurden in ein Zentrifugenröhrchen gefüllt und in einem Wasserbad bei 50 °C flüssig gehalten.

In die 10 ml flüssigen (50 °C) St. 1 Agar wurden 1000 *E. coli* Bakterien gegeben und nach dem Homogenisieren in autoklavierte Kunststoffringe unterschiedlicher Höhe gemäß nachfolgender Tabelle 5 pipettiert.

Tabelle 5: Menge St. 1 Agar mit *E. coli* pro Kunststoffring.

| Ringhöhe [mm] | Menge St. 1 Agar mit *E. coli* [$\mu$l] |
|---|---|
| 0,5 | 50 |
| 1,0 | 90 |
| 2,0 | 150 |
| 3,0 | 200 |

### Versuchsaufbau zur Inaktivierung von *E. coli* in Agar-Biofilm

Der resultierende Biofilm mit einem Durchmesser von 8 mm befand sich während des Versuchs zwischen einer Mikrodiamant-beschichteten Anode 12 aus Titan und einem Edelstahlblech als Kathode 16. Ein Titanstab 10 diente der Kontaktierung der Anode 12 bzw. Kathode 16, indem er händisch auf die beschichtete Anode 12 bzw. die Kathode 16 gedrückt wurde. Beim in Fig. 2 dargestellten Versuchsaufbau ist der Titanstab 10 mit dem Pluspol und das Edelstahlblech mit dem Minuspol einer Spannungsquelle verbunden. Unter einer sterilen Sicherheitswerkbank wurden Spannungen von 4,0 V, 4,2 V und 4,5 V zwischen 1 min und 6 min angelegt und der jeweils zugehörige Stromfluss gemessen.

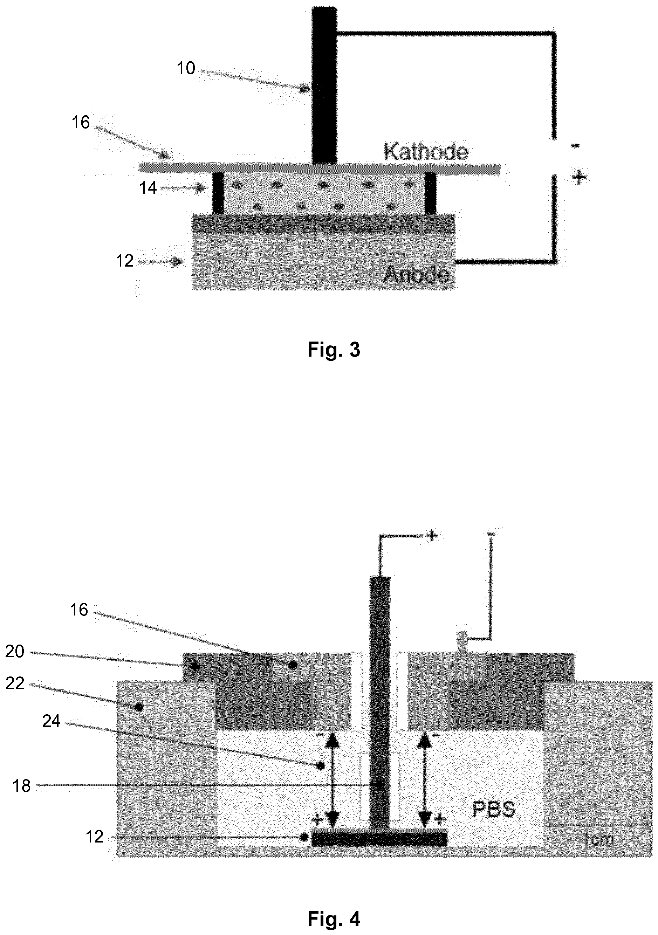

Um eine Diffusion des kathodisch gebildeten Wasserstoffgases von unten durch den Biofilm hindurch nach oben und damit eine Inaktivierung von *E. coli* durch Wasserstoff auszuschließen, wurden Anode 12 und Kathode 16 bei einigen Versuchen vertauscht. Der entsprechende Versuchsaufbau ist in Fig. 3 dargestellt.

### Quantifizierung der Bakterienkolonien

Anschließend erfolgte ein Heraustrennen der behandelten Biofilme aus den Ringen und deren Positionierung auf festen St. 1 Agar-Nährböden in Petrischalen. Bei 27 °C wurden sie circa 7 Tage inkubiert.

Die gebildeten Kolonien innerhalb der Biofilme waren mit dem bloßen Auge als dunkle Punkte zu erkennen und wurden bei 40-facher Vergrößerung im Lichtmikroskop ausgezählt.

### Inaktivierung von *Streptococcus gordonii*

### Initiale Adhäsion von *Streptococcus gordonii*

Die BDD-beschichteten Titanplättchen wurden initial bakteriell besiedelt. Als Nährmedium einer Vorkultur diente autoklaviertes Tryptone Soya Broth (TSB), das zusätzliche mit Hefeextrakt versetzt war. Die Bestandteile des Nährmediums sind in der nachfolgenden Tabelle 6 aufgelistet.

Tabelle 6: Inhaltsstoffe des Nährmediums Tryptone Soya Broth mit Hefeextrakt in 1 l dreifach destilliertem Wasser.

| Handelsname | Menge | Hersteller/ Art.-Nr. |
|---|---|---|
| Tryptone Soya Broth | 30,0 g | Fa. Oxoid LTD |
| Hefeextrakt | 3,0 g | Carl Roth GmbH/ 2363.3 |

50 ml des Nährmediums wurden mit 50 $\mu$l des oralen Bakteriums *S. gordonii* DSMZ 20568 in einem Erlenmeyerkolben angeimpft und für 18 h unter Rühren bei 37 °C aerob inkubiert. Anschließend wurde die resultierende Kultur in ein 50 ml Zentrifugenröhrchen überführt und bei 4 °C 15 min und 4000 g zentrifugiert. Der Überstand wurde abgegossen und das Pellet in 50 ml einer 50 mM Tris(hydroxymethyl)-aminomethan-Lösung, die mittels Salzsäure (HCl) auf pH 7,5 eingestellt wurde (TRIS HCl), resuspendiert. Anschließend wurden 25 ml TRIS HCl auf 500 ml mit dreifach destilliertem Wasser aufgefüllt. Die Schritte des Zentrifugierens und Resuspendierens wurden noch zweimal wiederholt. Beim letzten Resuspendieren verwendete man nur 20 ml des 50 mM TRIS Puffers und vortexte anschließend für 5 min. Mithilfe der verwendeten Pufferlösung wurde die resultierende Bakteriensuspension auf eine optische Dichte (OD) von 0,7 bei einer Wellenlänge von 600 nm eingestellt. Jedes der beschichteten Titanplättchen wurde jeweils mit der Beschichtung nach oben in eines der Näpfchen (Wells) einer Platte mit 6 Näpfchen (6-Well-Platte) der Firma Greiner Bio-One gelegt. Auf jedes der beschichteten Titanplättchen wurden 3 ml der Bakteriensuspension gegeben.

Eine initiale Adhäsion fand bei 37 °C in einem Inkubator bei Belüftungsstufe 3 und leichter Rotation mit 150 Umdrehungen pro Minute innerhalb von 5 h statt. Die besiedelten Plättchen wurden zweimal mit jeweils 3 ml phosphatgepufferter Salzlösung nach Dulbecco (PBS) gewaschen. Jedes Well wurde für den Versuch randvoll mit PBS gefüllt.

### Versuchsaufbau zur Inaktivierung von initial adhärierten *Streptococcus gordonii*

Der Versuchsaufbau ist in Fig. 4 dargestellt. Am Boden jedes der mit PBS gefüllten Wells 22 lagen die BDD-beschichteten und mit Bakterien besiedelten Titanplättchen, die im elektrochemischen Versuch als Anode 12 geschaltet wurden. Ein Titan-Aluminium-Stab 18 mit 2 mm Durchmesser kontaktierte die Anode 12. Die Kathode 16 aus Edelstahl mit dem Durchmesser 22 mm befand sich in einer runden Aussparung des Deckels 20 aus PVC, der an den Durchmesser des Wells 22 von 40 mm angepasst war. Durch ein Loch in der Kathode 16 wurde der Titan-Aluminium-Stab 18 zur Kontaktierung der Anode 12 hindurchgeführt. Teflon diente als Isolationsmaterial zwischen den beiden Elektroden. Der Titan-Aluminium-Stab 18 war mit dem Pluspol verbunden, während die Kathode 16 an den Minuspol einer Spannungsquelle angeschlossen war. Ein in Reihe geschaltetes Multimeter erfasste den Stromfluss bei angelegter Spannung. Durch Anlegen der Spannung entstand zwischen der Anode 12 und der Kathode 16 ein elektrisches Feld 24.

Im Anschluss an die Stimulation fand ein Waschvorgang mit 3 ml PBS statt, um die abgelösten Bakterien zu entfernen. Zur Bestimmung der Lebend-Totverteilung auf den Plättchen kam eine Lebend/Tot-Färbung der adhärierten Streptokokken mit Fluoreszenzfarbstoffen zum Einsatz. Dazu wurden der grüne Lebendfarbstoff SYTO9 und der rote Totfarbstoff Propidiumiodid aus dem Live/Dead BacLight Bactarial Viability Kit der Fa. Thermofisher Scientific eingesetzt. 3 ml einer Färbelösung, in der beide Farbstoffe jeweils 1:1000 mit PBS verdünnt sind, benetzten alle Plättchen. Da beide Farbstoffe lichtempfindlich sind, fand der Färbeprozess abgedunkelt statt. Die Einwirkzeit betrug circa 30 min.. Der Lebendfarbstoff bindet an die DNA von allen bakteriellen Zellen, während der Totfarbstoff nur in Bakterien mit zerstörter Membran eindringt und dabei den Lebendfarbstoff verdrängt. Danach ersetzte man die Färbelösung durch dieselbe Menge an 2,5%-igem Glutardialdehyd als Fixiermittel. Es handelt sich um eine hoch reaktive saure Lösung, die durch Reaktion mit Aminogruppen Proteine in der bakteriellen Membran quervernetzt und die Bakterienzellen somit abtötet. Vor der mikroskopischen Charakterisierung folgte nach mindestens 15 min Einwirkdauer ein Austausch der Fixierlösung gegen PBS.

In den ersten Versuchen wurden Spannungen zwischen 1,5 V und 3,5 V für 2 min bzw. 3 min angelegt. Aus diesen Ergebnissen wurde die Ladungsdichte, die zum Abtöten der adhärierten Bakterien notwendig ist, berechnet und diese in folgenden Versuchen gezielt eingestellt. Es handelte sich dabei um Flächenladungsdichten von 18 $(A^*s)/m^2$ bis 18.000 $(A^*s)/m^2$.

Jeder Versuch, bei dem Ladungsdichten eingestellt wurden, wurde dreimal in unabhängigen biologischen Replikaten durchgeführt. Es fanden Vergleiche zwischen Mikro- und Nanodiamantbeschichtung sowie zwischen Diamantbeschichtung und reinen Titanplättchen statt.

### Inaktivierung von humanen *Gingiva Fibroblasten*

Um den Einfluss der Bakterieninaktivierung mittels Radikalbildung an BDD-beschichteten Implantatoberflächen auf das umliegende Gewebe zu testen, diente ein Versuch mit sechs BDD-beschichteten Titanplättchen, wovon eines als unbehandelte Kontrolle diente.

Als Gewebezellen wurden humane *Gingiva Fibroblasten* aus dem Zahnfleisch verwendet. Diese Primärzellen sind aus unmittelbar zuvor noch intaktem Gewebe isoliert worden. Die Vorkultur wuchs innerhalb von drei Tagen in DMEM-Zellkulturmedium (Fa. Merck Millipore) mit den aus der nachfolgenden Tabelle 7 ersichtlichen Inhaltsstoffen, 10% fötalem Kälberserum und 1% Penicillin heran.

Tabelle 7: Inhaltsstoffe des DMEM-Zellkulturnährmediums in 0,5 l dreifach destilliertem Wasser.

| Handelsname | Menge |
|---|---|
| Natriumhydrogencarbonat | 3,7 g/l |
| D-Glukose | 4,5 g/l |
| L-Glutamin | 0,6 g/l |

Auf jedem der BDD-beschichtetem Titanplättchen wurden 50.000 Zellen in 120 $\mu$l einer Zellsuspension aus-

gesät. Die Zellen befanden sich hierbei in der achten Passage. Die Plättchen wurden 2 h bei 37 °C und 5% $CO_2$ inkubiert. Der pH-Wert des Zellkulturmediums betrug dabei circa 7,7. Nach der Adhäsion der Fibroblasten erfolgte die Benetzung jedes der sechs Plättchen in den Wells 22 mit 3 ml DMEM-Zellkulturmedium sowie eine Inkubation für 24 h. Es schloss sich die Behandlung in PBS mit dem in Fig. 4 dargestellten Versuchsaufbau und den gleichen Ladungsdichten, wie bei initialer Adhäsion und Biofilm von *S. gordonii* an. Pro Well fand danach ein Ersetzen des PBS mit 3 ml der Färbelösung statt. Darin sind beide Farbstoffe jeweils 1:1000 mit PBS verdünnt. Als Lebendfarbstoff diente Calcein der Fa. Invitrogen, als Totfarbstoff wurde Propidiumiodid der Fa. SIGMA eingesetzt. Zur Auswertung des Versuchs dienten fünf fotografische Aufnahmen pro Plättchen mit dem Mikroskop Axio Scope A1 der Fa. Zeiss in 200facher Vergrößerung.

**Ergebnisse und Diskussion**

Alle Ergebnisse sind über der elektrischen Ladungsdichte als Maß für die entstehenden bakteriziden Oxidationsmittel aufgetragen. Diese wurde entweder gezielt im Versuch eingestellt oder anschließend berechnet. Dazu mussten der gemessene Strom bei anliegender Spannung, die zugehörige Behandlungsdauer und das Volumen des Biofilms bzw. die Fläche der adhärierten Bakterien bekannt sein.

In einem Volumen wird die Ladungsverteilung als Raumladungsdichte $\varrho$ bezeichnet.

$$[\varrho] = 1 \frac{As}{m^3}$$

Die gleichmäßige Verteilung der Ladung auf einer Fläche wird als Flächenladungsdichte $\sigma$ beschrieben.

$$[\sigma] = 1 \frac{As}{m^2}$$

**Inaktivierung von *Escherichia coli* im Agar-Biofilm**

**E. coli-Inaktivierung in verschiedenen Biofilm-Volumen**

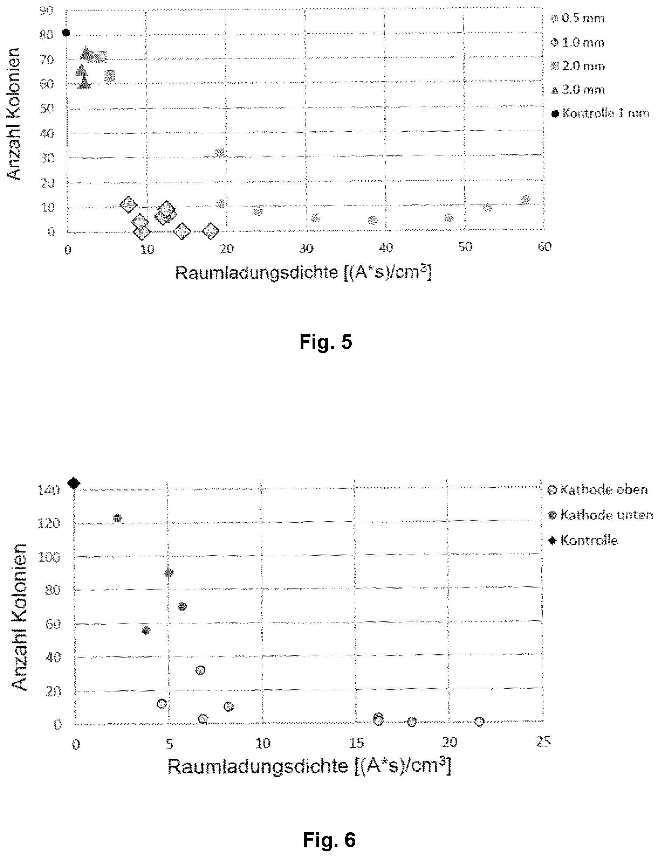

Das Diagramm in Fig. 5 veranschaulicht einen Versuch, bei dem jeder Biofilm für 2 min mit 4,2 V bei oben positionierter Kathode 16 gemäß dem in Fig. 3 dargestellten Versuchsaufbau belastet wurde. Bei jedem Punkt in der Grafik handelt es sich um die gemittelte Anzahl der Kolonien aus drei Stellen in einem Biofilm. Es ist eindeutig ersichtlich, dass bei dieser konstanten Spannung und Behandlungszeit in dünneren Biofilmen höhere Raumladungsdichten auftreten als in den dickeren und damit dort mehr Kolonien inaktiviert werden. Innerhalb gleicher Biofilmhöhen ergeben sich aufgrund von unterschiedlichen Kontaktwiderständen und damit verbundenen schwankenden Stromstärken, voneinander abweichende Raumladungsdichten. In den 2 mm und 3 mm hohen Biofilmen unterscheiden sich die Raumladungsdichten sowie die Anzahl der Kolonien kaum, während die dünneren Biofilme deutlich weniger Kolonien beinhalten. Ein vollständiges Absterben findet ab circa 10 (A*s)/$cm^3$ statt. Das Volumen der 0,5 mm Biofilme entspricht 0,025 $cm^3$, bei 1 mm sind es 0,05 $cm^3$, bei 2 mm sind es 0,1 $cm^3$ und bei 3 mm beträgt das Volumen 0,16 $cm^3$.

Fig. 5 zeigt die Abhängigkeit der Anzahl der *E. coli* Kolonien von der Raumladungsdichte bei konstanter Spannung von 4,2 V für verschiedene Biofilmvolumina. Insgesamt sinkt die Anzahl der Bakterienkolonien mit höheren Raumladungsdichten. In den dünneren Biofilmen werden die Bakterien stärker inaktiviert, da dort höhere Raumladungsdichten erreicht werden.

**Vergleich zweier unterschiedlicher Anordnungen der Kathode**

Um zu überprüfen, ob die kathodische Wasserstoffentwicklung die Bakterieninaktivierung durch Hydroxylradikale und andere Oxidationsmittel verstärkt, wurde die Position der Kathode variiert, wie in Fig. 2 und Fig. 3 veranschaulicht.

Es wurde erwartet, dass atomarer Wasserstoff bei unten befindlicher Kathode durch den Biofilm nach oben diffundiert und dabei eventuell Bakterien abtöten kann. Die Ergebnisse zeigen jedoch in diesem Fall eine geringere Inaktivierung von *E. coli* durch unzureichende Raumladungsdichten. Wird der Aufbau umgedreht und die Kathode oben positioniert, sind die Kontaktwiderstände geringer, damit die Raumladungsdichten höher und mehr Bakterien sterben. Dies ist bei einem Versuch mit 4,2 V und 1 mm dicken Agar-Biofilmen in Fig. 6 dargestellt.

Fig. 6 zeigt die Anzahl der *E. coli* Kolonien in Abhängigkeit von der Raumladungsdichte. Dabei wurde der Versuchsaufbau gemäß Fig. 2 und Fig. 3 zur Inaktivierung von *E. coli* bei 4,2 V in 1 mm dicken Agar-Biofilmen für 1 und 3 min verglichen. Es zeigte sich, dass bei unten angeordneter Kathode keine verstärkte Bakterieninaktivierung durch den gebildeten Wasserstoff stattfand.

**Zusammenfassung zur Inaktivierung von *Escherichia coli* im Agar-Biofilm**

Im Allgemeinen zeigt sich bei allen Versuchen mit unterschiedlichen Biofilmvolumina, dass mit höheren Raumladungsdichten mehr Bakterienkolonien inaktiviert wurden und die Raumladungsdichte definitionsgemäß umgekehrt proportional zur Biofilmhöhe war.

Es konnte gezeigt werden, dass *E. coli* sich durch elektrochemische Behandlung mit Diamantanoden vollständig inaktivieren lässt. Bei 4,0 V bzw. 4,2 V ist dazu die Mindest-Ladungsdichte von 2 (A*s)/cm$^3$ bzw. 10 (A*s)/cm$^3$ für 2 min in 1 mm dicken Biofilmen notwendig.

Eine Inaktivierung von *E. coli* durch kathodisch gebildeten Wasserstoff im Versuchsaufbau gemäß Fig. 2 konnte nicht nachgewiesen werden.

Eine Abtötung von *E. coli* durch eine hohe Temperaturentwicklung während des elektrochemischen Versuchs wurde ebenfalls untersucht und konnte ausgeschlossen werden.

**Inaktivierung von initial adhäriertem *Streptococcus gordonii***

Die folgenden Ergebnisse beziehen sich auf elektrochemische Versuche in einem flüssigen Elektrolyten. Der Versuchsaufbau ist in Fig. 4 gezeigt.

Aufgrund einer sehr geringen Höhe der vereinzelt adhärierten *S. gordonii* sowie der Biofilme, die weniger als 30 μm beträgt, wurde hier die Lebend-Totverteilung in den Diagrammen auf die Flächenladungsdichte bezogen.

**Ermittlung der Spannung zur Bakterieninaktivierung**

Es wurde der Spannungsbereich bestimmt, bei dem eine Oxidationsmittel-bedingte Desinfektion der BDD-beschichteten und durch bakterielle Adhäsion mit *S. gordonii* besiedelten Oberfläche der Titanplättchen zu beobachten war.

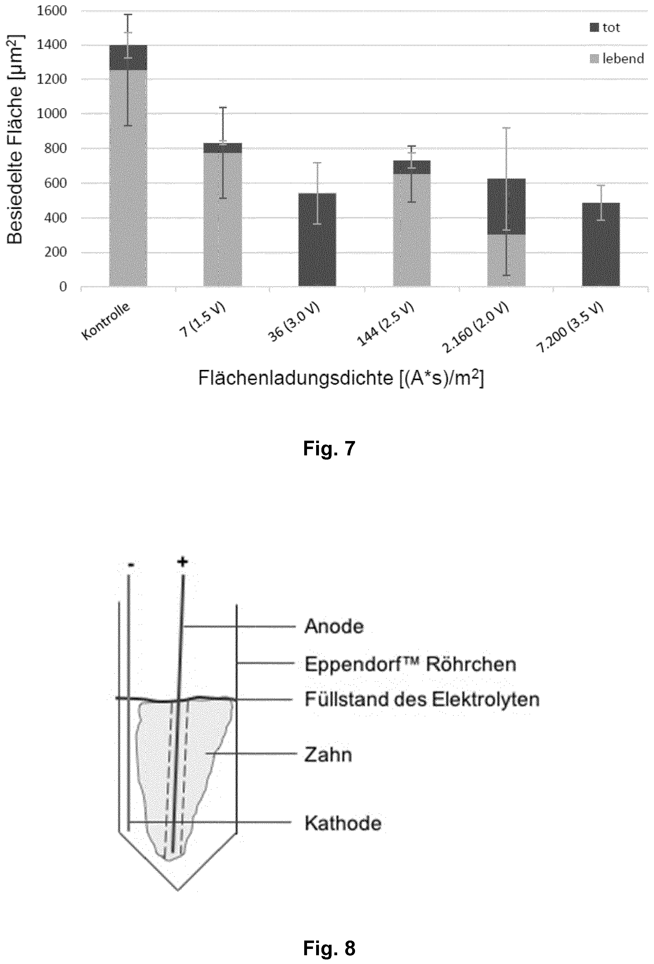

In Fig. 7 ist die von *S. gordonii* durch initiale Adhäsion auf BDD-beschichteten Titanplättchen besiedelte mittlere Fläche pro Probe nach Anlegen verschiedener Spannungen in Abhängigkeit von der berechneten Flächenladungsdichte in (A*s)/m$^2$ und der angelegten Spannung dargestellt. Alle fünf Proben werden für 2 min behandelt. An die besiedelte Kontrolle wurde keine Spannung angelegt. Die Standardabweichungen zeigen die Schwankung der fünf Messpunkte innerhalb einer Probe. Auf der unbehandelten Kontrolle befinden sich nur wenige tote Zellen, die auf natürliche Weise abgestorben sind. Bei den mit 1,5 V und 2,5 V behandelten Titanplättchen ist der Totanteil vergleichbar mit demjenigen der Kontrolle. Der Lebendanteil auf diesen Proben ist jedoch deutlich geringer als der der Kontrolle. Vermutlich fanden dort weniger Keime Überlebensbedingungen zur Besiedlung vor oder die Keime wurden beim Waschen leichter abgespült. Einen sehr hohen Flächenanteil toter Bakterien zeigen die Proben auf, an die 3,0 V und 3,5 V angelegt wurde. Bei diesen Spannungen wurden nahezu alle oralen Keime inaktiviert.

Der Vergleich verschiedener Spannungen an mit Bor-dotiertem Mikrodiamant beschichteten Plättchen (K571) weist darauf hin, dass zwischen 2,5 V und 3,0 V der Übergang von lebenden zu toten *S. gordonii* Keimen stattfindet.

Nicht dargestellte Versuche mit Bor-dotierten Mikro- und Nanodiamantbeschichtungen zeigten keinen entscheidenden Einfluss der Diamantstruktur auf die elektrochemische Inaktivierung von S. gordonii. Die Morphologie des Diamants wirkt sich lediglich auf die Anzahl der adhärierten Bakterien aus, jedoch nicht auf deren inaktivierten Anteil.

**Einfluss der oxidierenden Produkte auf humane *Gingiva Fibroblasten***

Ein Versuch mit menschlichen Fibroblasten des Zahnfleisches unter gleichen Bedingungen wie bei den mit *S. gordonii* durchgeführten Versuchen, zeigt keine Abtötung der eukaryotischen Zellen. Unter Behandlung mit den höchsten Ladungsdichten sind ausschließlich grün angefärbte lebendige Fibroblasten zu erkennen. Daraus lässt sich schließen, dass eine elektrochemische Oberflächendesinfektion mit Spannungen von bis zu 3,5 V und Flächenladungsdichten von 18.000 (A*s)/m$^2$ im Mundraum prinzipiell möglich ist.

**Fazit**

Die Erfinder konnten zeigen, dass mit einer Bor-dotierten Diamantbeschichtung auf einer Anode sowohl gramnegative *Escherichia coli* Bakterien als auch grampositive *Streptococcus gordonii* Spezies vollständig elektrochemisch inaktiviert werden können. Damit bieten BDD-beschichtete Implantate die Möglichkeit Entzündungsreaktionen elektrochemisch vorzubeugen und Infektionen zu bekämpfen.

Eine elektrochemische Therapie einer mikrobiellen Infektion an einer BDD-beschichteten Implantatoberfläche mit verhältnismäßig geringer Spannung und Ladungsdichte ist bei der dazu erforderlichen kurzen Behandlungszeit problemlos für den menschlichen oder tierischen Körper verträglich. Bei Bedarf kann die Behandlung jederzeit wiederholt oder unterbrochen werden.

Da es bei der Therapie direkt an der Diamantanode zur Entstehung zahlreicher Oxidationsmittel kommt, können Bakterien an der Implantatoberfläche direkt und effektiv inaktiviert werden. Fibroblasten haben experimentell nicht negativ auf diese Behandlung reagiert.

**Inaktivierung von *Staphylococcus epidermidis* und *Bacillus subtilis* in einem ausgebohrten Wurzelkanal eines menschlichen Zahns**

Menschliche extrahierte Zähne mit einem ausgebohrten Wurzelkanal wurden von einem Zahnarzt erhalten. Die Zähne wurden zunächst für mindestens 20 Stunden entweder in einer *Staphylococcus epidermidis* oder *Bacillus subtilis* enthaltenden physiologischen Kochsalzlösung inkubiert. Dabei wurden die Wurzelkanäle mit den jeweiligen Bakterien besiedelt. Anschließend wurden die Zähne mit physiologischer Kochsalzlösung gespült und in physiologischer Kochsalzlösung als Elektrolyt in dem aus Fig. 8 ersichtlichen Versuchsaufbau angeordnet. Die Anode bestand dabei aus einem mit Bor-dotiertem Diamant beschichteten Niobdraht und die Kathode aus Stahl. Es wurde eine Spannung im Bereich von 5 bis 9 Volt angelegt, so dass die in den Figuren angegebenen Strommengen in der jeweils angegeben Zeit der Behandlung geflossen sind.

Nach der jeweils angegebenen Zeit der Behandlung wurde der jeweilige Zahn gespalten und die dadurch erhaltenen die Oberfläche des Wurzelkanals umfassenden inneren Spaltflächen wurden auf einem St.1 Agar-Nährboden mehrfach eingedrückt und schließlich mit dieser Fläche auf dem Agar gelagert. Der Agar-Nährboden wurde anschließend bei 27 Grad für 1-2 Tage inkubiert. Bakterienwachstum war durch die Bildung von Kolonien zu erkennen. Die Kolonien waren mit dem bloßen Auge als Punkte zu erkennen und wurden subjektiv in kein, mäßiges, mittleres und starkes bakterielles Wachstum kategorisiert.

**Inaktivierung von *Staphylococcus epidermidis***

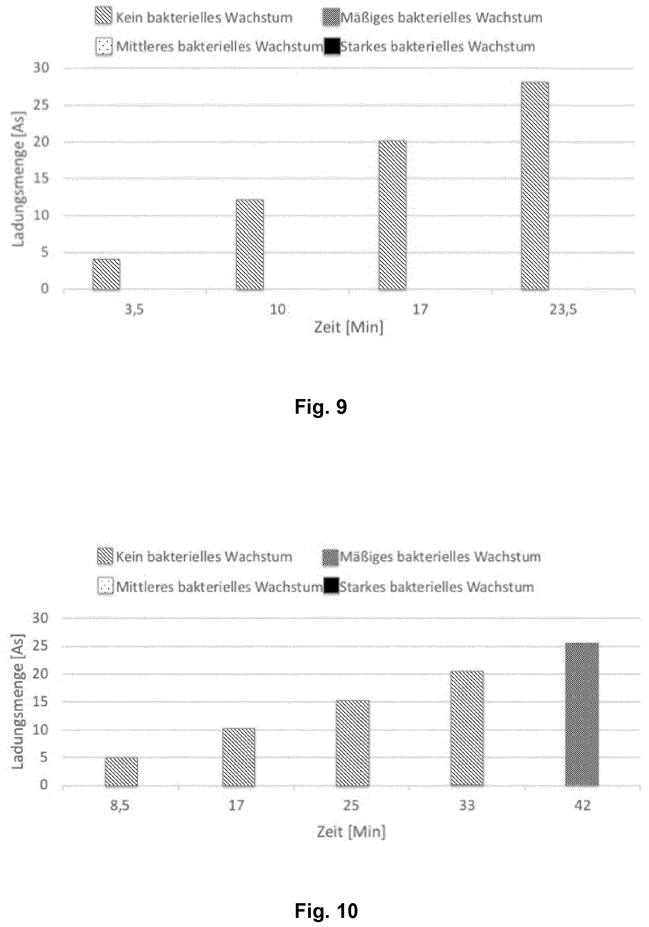

Die aus Fig. 9 ersichtlichen Ergebnisse zeigen, dass bereits bei einer Strommenge von 4 As, entsprechend einer Behandlungszeit von 3,5 Minuten, eine vollständige Sterilisation der inneren Wurzelkanaloberfläche erreicht wurde. Auf keiner der Agar-Nährböden zeigte sich ein Bakterienwachstum.

**Inaktivierung von *Bacillus subtilis***

Die aus Fig. 10 ersichtlichen Ergebnisse zeigen, dass bereits bei einer Strommenge von 5 As, entsprechend einer Behandlungszeit von 8,5 Minuten, eine vollständige Sterilisation der inneren Wurzelkanaloberfläche festgestellt wurde. Ein sich nach einer Behandlungszeit von 42 Minuten und einer Strommenge von 25 As zeigendes mäßiges bakterielles Wachstum kann dadurch verursacht worden sein, dass *Bacillus subtilis* in der Lage ist, Sporen zu bilden und diese Sporen möglicherweise in den Dentinkanälchen der Zahnwurzel enthalten waren. Es wurde weiterhin festgestellt, dass sich bei einer Sporenbildung die zur vollständigen Sterilisation erforderliche Ladungsmenge mehr als verhundertfachen kann.

**Ergebnisse und Diskussion**

Die am extrahierten Zahn durchgeführten Versuche zeigen, dass sich eine mit Bor-dotiertem Diamant beschichtete Anode gut zum Desinfizieren eines Wurzelkanals in verhältnismäßig kurzer Zeit eignet. Eine solche Anode, beispielsweise in Form eines mit Bor-dotiertem Diamant beschichteten Drahtes, kann dazu bei einer Zahnbehandlung in einen eröffneten Wurzelkanal eingeführt und unter Strom gesetzt werden. Die dazu erforderliche Kathode kann in der Nähe des zu behandelnden Zahns innerhalb der Mundhöhle angeordnet werden. Der in den Wurzelkanal einzuführende beschichtete Draht stellt ein Implantat im Sinne der Erfindung dar.

Bezugszeichenliste

10 Titanstab
12 Anode
16 Kathode

18    Titan-Aluminium-Stab
20    Deckel
22    Well
24    elektrisches Feld

**Patentansprüche**

1. An einem Implantat aus einem Metall oder mit einer Oberfläche aus einem Metall erzeugte oxidierende Produkte mit antimikrobieller Wirkung zur Verwendung bei einer therapeutischen Behandlung, wobei das Implantat auf dessen/der Oberfläche eine Beschichtung mit polykristallinem dotiertem elektrisch leitfähigem Diamant aufweist, wobei die therapeutische Behandlung eine Therapie einer mikrobiellen Infektion durch Bakterien am Implantat eines menschlichen oder tierischen Körpers ist, wobei die therapeutische Behandlung die Erzeugung der Produkte mit antimikrobieller Wirkung direkt am Implantat umfasst, wobei die Produkte mit antimikrobieller Wirkung $C_2O_6^{2-}$, $S_2O_8^{2-}$ und/oder $P_2O_8^{4-}$-Ionen sind,

   - wobei das Implantat in einem elektrochemischen System in dem Körper als Anode (12) geschaltet ist, wobei das elektrochemische System neben der Anode (12) eine Kathode (16), eine jeweils mit der Anode und der Kathode elektrisch leitend verbundene Stromquelle und einen eine Körperflüssigkeit umfassenden oder aus einer Körperflüssigkeit bestehenden Elektrolyten umfasst oder aus der Anode (12), einer Kathode (16), einer jeweils mit der Anode und der Kathode elektrisch leitend verbundenen Stromquelle und einem eine Körperflüssigkeit umfassenden oder aus einer Körperflüssigkeit bestehenden Elektrolyten besteht oder
   - wobei das Implantat innerhalb eines elektrischen Feldes angeordnet ist, wodurch durch Induktion auf dem Implantat an einer ersten Stelle eine negative Ladung und an einer zweiten Stelle eine positive Ladung induziert wird, wodurch die erste Stelle in einem elektrochemischen System zur Anode (12) und die zweite Stelle in dem elektrochemischen System zur Kathode (16) wird, wobei das elektrochemische System neben dem Implantat einen eine Körperflüssigkeit umfassenden oder aus einer Körperflüssigkeit bestehenden Elektrolyten umfasst oder aus dem Implantat und einem eine Körperflüssigkeit umfassenden oder aus einer Körperflüssigkeit bestehenden Elektrolyten besteht.

2. An dem Implantat erzeugte Produkte mit antimikrobieller Wirkung nach Anspruch 1 zur Verwendung nach Anspruch 1, wobei der Diamant mit Bor oder Phosphor dotiert ist.

3. An dem Implantat erzeugte Produkte mit antimikrobieller Wirkung nach einem der vorhergehenden Ansprüche zur Verwendung nach Anspruch 1, wobei die mikrobielle Infektion eine bakterielle Infektion oder eine Infektion durch einen Pilzbefall ist.

4. An dem Implantat erzeugte Produkte mit antimikrobieller Wirkung nach einem der vorhergehenden Ansprüche zur Verwendung nach Anspruch 1, wobei das Metall Titan oder eine Titan enthaltende Legierung ist.

5. An dem Implantat erzeugte Produkte mit antimikrobieller Wirkung nach Anspruch 4 zur Verwendung nach Anspruch 1, wobei die Legierung Ti-6Al-4V, Ti-6Al-7Nb oder eine sonstige Legierung ist, die neben Titan Aluminium und/oder Niob und/oder Eisen und/oder Molybdän enthält.

6. An dem Implantat erzeugte Produkte mit antimikrobieller Wirkung nach einem der vorhergehenden Ansprüche zur Verwendung nach Anspruch 1, wobei die Beschichtung eine Beschichtung ist, die durch heißdraht-aktivierte chemische Gasphasenabscheidung (hot filament chemical vapor deposition = HFCVD) oder durch mikrowellenplasmaunterstützte chemische Gasphasenabscheidung (microwave plasma-assisted chemical vapor deposition = MPCVD) oder durch eine sonstige chemische Gasphasenabscheidung (chemical vapor deposition = CVD) an der Oberfläche des Implantats in einer eine borhaltige Verbindung enthaltenden Gasphase erzeugt ist.

7. An dem Implantat erzeugte Produkte mit antimikrobieller Wirkung nach einem der vorhergehenden Ansprüche zur Verwendung nach Anspruch 1, wobei das Metall Titan oder die Titan enthaltende Legierung ist und sich zwischen dem Titan oder der Legierung und der Beschichtung eine Zwischenschicht aus Titancarbid befindet.

8. An dem Implantat erzeugte Produkte mit antimikrobieller Wirkung nach Anspruch 7 zur Verwendung nach Anspruch 1, wobei die Zwischenschicht eine Schichtdicke von höchstens 3 $\mu$m aufweist.

**9.** An dem Implantat erzeugte Produkte mit antimikrobieller Wirkung nach Anspruch 7 oder 8 zur Verwendung nach Anspruch 1, wobei die Zwischenschicht eine Schichtdicke von mindestens 100 nm aufweist.

**10.** An dem Implantat erzeugte Produkte mit antimikrobieller Wirkung nach einem der vorhergehenden Ansprüche zur Verwendung nach Anspruch 1, wobei das Implantat ein von außerhalb des Körpers ohne chirurgischen Eingriff zugängliches Zahn- oder Kieferimplantat ist und für die Therapie von außerhalb des Körpers elektrisch kontaktiert wird oder wobei das Implantat in ein Inneres des Körpers eingesetzt ist und ein elektrischer Leiter, insbesondere ein elektrisch leitender Draht, vom Implantat auf eine Außenseite des Körpers geführt ist und für die Therapie auf der Außenseite elektrisch kontaktiert wird oder vom Implantat zu einer Kontaktstelle unter der Haut geführt ist, die für die Therapie von außen durch eine Punktion elektrisch kontaktiert wird oder vom Implantat über einen elektrischen Gleichrichter zu einer, insbesondere nicht mehr als 3 cm unter der Haut angeordneten, Spule geführt und mit dieser elektrisch leitend verbunden ist, wobei die Spule weiterhin über den elektrischen Gleichrichter elektrisch leitend mit der Kathode (16) verbunden ist, wobei die Kathode (16) ebenfalls im Körper angeordnet ist, wobei für die Therapie in der Spule von außerhalb des Körpers durch Induktion ein Stromfluss induziert wird, durch welchen die Kathode (16) und die Anode (12) unter Strom gesetzt werden.

**11.** An dem Implantat erzeugte Produkte mit antimikrobieller Wirkung nach einem der Ansprüche 1 bis 9 zur Verwendung nach Anspruch 1, wobei das elektrische Feld zwischen mindestens zwei Platten eines Kondensators oder durch mindestens eine elektrische Spule aufgebaut ist oder zwischen einer weiteren Anode und einer weiteren Kathode innerhalb des elektrochemischen Systems aufgebaut ist, wobei das elektrochemische System zusätzlich neben der weiteren Anode und der weiteren Kathode eine mit der weiteren Anode und der weiteren Kathode elektrisch leitend verbundene Stromquelle umfasst oder zusätzlich aus der weiteren Anode und der weiteren Kathode sowie einer mit der weiteren Anode und der weiteren Kathode elektrisch leitend verbundenen Stromquelle besteht.

**12.** An dem Implantat erzeugte Produkte mit antimikrobieller Wirkung nach Anspruch 11 zur Verwendung nach Anspruch 1, wobei der Elektrolyt in dem elektrochemischen System eine elektrisch leitende Hilfsflüssigkeit umfasst oder aus der Körperflüssigkeit und der elektrisch leitenden Hilfsflüssigkeit besteht, wobei die elektrisch leitende Hilfsflüssigkeit jeweils eine einen elektrischen Kontakt der weiteren Anode und der weiteren Kathode mit der Körperflüssigkeit herstellende Flüssigkeit ist.

**13.** An dem Implantat erzeugte Produkte mit antimikrobieller Wirkung nach einem der vorhergehenden Ansprüche zur Verwendung nach Anspruch 1, wobei das Implantat innerhalb eines geschlossenen Bereichs des Körpers angeordnet ist, wobei eine an das elektrochemische System angelegte Spannung so gewählt wird, dass die Menge der an der Anode entstehenden gasförmigen weiteren Produkte so gering ist, dass sich diese unmittelbar nach deren Entstehen in der Körperflüssigkeit lösen.

**14.** An dem Implantat erzeugte Produkte mit antimikrobieller Wirkung nach einem der vorhergehenden Ansprüche zur Verwendung nach Anspruch 1, wobei die mikrobielle Infektion eine mit einer Entzündungsreaktion einhergehende mikrobielle Infektion ist.

**15.** An dem Implantat erzeugte Produkte mit antimikrobieller Wirkung nach einem der vorhergehenden Ansprüche zur Verwendung nach Anspruch 1, wobei das Implantat eine Endoprothese aus einem Metall ist.

**Claims**

**1.** Oxidizing products having antimicrobial action that have been produced on an implant made of a metal or having a surface made of a metal for use in a therapeutic treatment, wherein the implant has, on its/the surface, a coating with polycrystalline doped electrically conductive diamond, wherein the therapeutic treatment is a therapy of a microbial infection caused by bacteria on the implant in a human or animal body, wherein the therapeutic treatment comprises the generation of the products having antimicrobial action directly on the implant, wherein the products having antimicrobial action are $C_2O_6^{2-}$, $S_2O_8^{2-}$ and/or $P_2O_8^{4-}$ ions,

- wherein the implant is connected as anode (12) in an electrochemical system in the body, wherein the electrochemical system comprises, in addition to the anode (12), a cathode (16), a power source connected in an electrically conductive manner to the anode and to the cathode, and an electrolyte comprising or consisting of a body fluid, or consists of the anode (12), a cathode (16), a power source connected in an electrically conductive manner to the anode and to the cathode, and an electrolyte comprising or consisting of a body fluid, or

- wherein the implant is disposed within an electrical field, by means of which a negative charge is induced at a first site and a positive charge at a second site by induction on the implant, by means of which the first site becomes the anode (12) in an electrochemical system and the second site becomes the cathode (16) in the electrochemical system, wherein the electrochemical system comprises, in addition to the implant, an electrolyte comprising or consisting of a body fluid or consists of the implant and an electrolyte comprising or consisting of a body fluid.

2. Products having antimicrobial action that have been produced on the implant as claimed in claim 1 for use as claimed in claim 1, wherein the diamond has been doped with boron or phosphorus.

3. Products having antimicrobial action that have been produced on the implant as claimed in either of the preceding claims for use as claimed in claim 1, wherein the microbial infection is a bacterial infection or a fungal infection.

4. Products having antimicrobial action that have been produced on the implant as claimed in any of the preceding claims for use as claimed in claim 1, wherein the metal is titanium or a titanium-containing alloy.

5. Products having antimicrobial action that have been produced on the implant as claimed in claim 4 for use as claimed in claim 1, wherein the alloy is Ti-6AI-4V, Ti-6AI-7Nb or another alloy containing, in addition to titanium, aluminum and/or niobium and/or iron and/or molybdenum.

6. Products having antimicrobial action that have been produced on the implant as claimed in any of the preceding claims for use as claimed in claim 1, wherein the coating is a coating produced on the surface of the implant in a gas phase containing a boron containing compound by hot filament chemical vapor deposition (HFCVD) or by microwave plasma-assisted chemical vapor deposition (MPCVD) or by another kind of chemical vapor deposition (CVD).

7. Products having antimicrobial action that have been produced on the implant as claimed in any of the preceding claims for use as claimed in claim 1, wherein the metal is titanium or the titanium-containing alloy and there is an interlayer of titanium carbide between the titanium or the alloy and the coating.

8. Products having antimicrobial action that have been produced on the implant as claimed in claim 7 for use as claimed in claim 1, wherein the interlayer has a layer thickness of not more than 3 $\mu$m.

9. Products having antimicrobial action that have been produced on the implant as claimed in claim 7 or 8 for use as claimed in claim 1, wherein the interlayer has a layer thickness of at least 100 nm.

10. Products having antimicrobial action that have been produced on the implant as claimed in any of the preceding claims for use as claimed in claim 1, wherein the implant is a dental implant or jaw implant which is accessible from outside the body without surgical intervention and is electrically contacted for the therapy from outside the body, or wherein the implant has been implanted within the body and an electrical conductor, especially an electrically conductive wire, is routed from the implant to an outside of the body and is electrically contacted on the outside for the therapy or is routed from the implant to a contact site beneath the skin that is electrically contacted for the therapy from the outside through a puncture or is routed from the implant via an electrical rectifier to a coil, especially disposed not more than 3 cm beneath the skin, and is connected thereto in an electrically conductive manner, wherein the coil additionally has electrically conductive connection via the electrical rectifier to the cathode (16), wherein the cathode (16) is likewise disposed within the body, wherein, for the therapy, a flow of current is induced in the coil by induction from outside the body, by means of which the cathode (16) and the anode (12) are energized.

11. Products having antimicrobial action that have been produced on the implant as claimed in any of claims 1 to 9 for use as claimed in claim 1, wherein the electrical field is built up between at least two plates of a capacitor or by means of at least one electrical coil or is built up between a further anode and a further cathode within the electrochemical system, wherein the electrochemical system additionally comprises, in addition to the further anode and the further cathode, a power source connected in an electrically conductive manner to the further anode and the further cathode or additionally consists of the further anode and the further cathode and a power source connected in an electrically conductive manner to the further anode and the further cathode.

12. Products having antimicrobial action that have been produced on the implant as claimed in claim 11 for use as claimed in claim 1, wherein the electrolyte in the electrochemical system comprises an electrically conductive auxiliary fluid or consists of the body fluid and the electrically conductive auxiliary fluid, wherein the electrically conductive auxiliary fluid in each case is a liquid that establishes electrical contact of the further anode and the further cathode with the body fluid.

13. Products having antimicrobial action that have been produced on the implant as claimed in any of the preceding claims for use as claimed in claim 1, wherein the implant is disposed within a closed region of the body, wherein a voltage applied to the electrochemical system is chosen such that the amount of gaseous further products formed at the anode is sufficiently low that they dissolve directly in the body fluid after they have formed.

14. Products having antimicrobial action that have been produced on the implant as claimed in any of the preceding claims for use as claimed in claim 1, wherein the microbial infection is a microbial infection associated with an inflammation reaction.

15. Products having antimicrobial action that have been produced on the implant as claimed in any of the preceding claims for use as claimed in claim 1, wherein the implant is an endoprosthesis made of a metal.


**Revendications**

1. Produits oxydants à action antimicrobienne, générés au niveau d'un implant en un métal ou avec une surface en un métal, destinés à être utilisés lors d'un traitement thérapeutique, dans lesquels l'implant présente sur sa/la surface un enrobage avec du diamant dopé polycristallin électriquement conducteur, dans lesquels le traitement thérapeutique est une thérapie d'une infection microbienne par des bactéries au niveau de l'implant d'un corps humain ou animal, dans lesquels le traitement thérapeutique comprend la génération des produits à action antimicrobienne directement au niveau de l'implant, dans lesquels les produits à action antimicrobienne sont des ions $C_2O_6^{2-}$, $S_2O_8^{2-}$ et/ou $P_2O_8^{4-}$,

- dans lesquels l'implant est monté dans un système électrochimique dans le corps en tant qu'anode (12), dans lesquels le système électrochimique comprend outre l'anode (12) une cathode (16), une source de courant connectée à chaque fois à l'anode et la cathode de manière électriquement conductrice et un électrolyte comprenant un fluide corporel ou constitué d'un fluide corporel, ou se compose de l'anode (12), d'une cathode (16), d'une source de courant connectée à chaque fois à l'anode et la cathode de manière électriquement conductrice et d'un électrolyte comprenant un fluide corporel ou constitué d'un fluide corporel, ou
- dans lesquels l'implant est disposé au sein d'un champ électrique, moyennant quoi une charge négative est induite par induction sur l'implant à un premier endroit et une charge positive à un second endroit, moyennant quoi le premier endroit devenant une anode (12) dans un système électrochimique et le second endroit devenant une cathode (16) dans le système électrochimique, dans lesquels le système électrochimique comprend outre l'implant un électrolyte comprenant un fluide corporel ou constitué d'un fluide corporel ou se compose de l'implant et d'un électrolyte comprenant un fluide corporel ou constitué d'un fluide corporel.

2. Produits à action antimicrobienne générés au niveau de l'implant selon la revendication 1 destinés à être utilisés selon la revendication 1, dans lesquels le diamant est dopé avec du bore ou du phosphore.

3. Produits à action antimicrobienne générés au niveau de l'implant selon une des revendications précédentes destinés à être utilisés selon la revendication 1, dans lesquels l'infection microbienne est une infection bactérienne ou une infection par un champignon.

4. Produits à action antimicrobienne générés au niveau de l'implant selon une des revendications précédentes destinés à être utilisés selon la revendication 1, dans lesquels le métal est le titane ou un alliage contenant du titane.

5. Produits à action antimicrobienne générés au niveau de l'implant selon la revendication 4 destinés à être utilisés selon la revendication 1, dans lesquels l'alliage est Ti-6Al-4V, Ti-6Al-7Nb ou un autre alliage qui contient outre du titane de l'aluminium et/ou du niobium et/ou du fer et/ou du molybdène.

6. Produits à action antimicrobienne générés au niveau de l'implant selon une des revendications précédentes destinés à être utilisés selon la revendication 1, dans lesquels l'enrobage est un enrobage qui est généré par dépôt chimique en phase vapeur assisté par filament chaud (hot filament chemical vapor deposition = HFCVD) ou par dépôt chimique en phase vapeur assisté par plasma micro-ondes (microwave plasma-assisted chemical vapor deposition = MPCVD) ou par un autre dépôt chimique en phase vapeur (chemical vapor deposition = CVD) à la surface de l'implant dans une phase vapeur contenant un composé contenant du bore.

7. Produits à action antimicrobienne générés au niveau de l'implant selon une des revendications précédentes destinés à être utilisés selon la revendication 1, dans lesquels le métal est le titane ou un alliage contenant du titane et une couche intermédiaire en carbure de titane se trouve entre le titane ou l'alliage et l'enrobage.

8. Produits à action antimicrobienne générés au niveau de l'implant selon la revendication 7 destinés à être utilisés selon la revendication 1, dans lesquels la couche intermédiaire présente une épaisseur de couche de 3 $\mu$m maximum.

9. Produits à action antimicrobienne générés au niveau de l'implant selon la revendication 7 ou 8 destinés à être utilisés selon la revendication 1, dans lesquels la couche intermédiaire présente une épaisseur de couche d'au moins 100 nm.

10. Produits à action antimicrobienne générés au niveau de l'implant selon une des revendications précédentes destinés à être utilisés selon la revendication 1, dans lesquels l'implant est un implant dentaire ou maxillaire accessible de l'extérieur du corps sans intervention chirurgicale et fait l'objet d'un contact électrique de l'extérieur du corps pour la thérapie ou dans lesquels l'implant est inséré dans une partie intérieure du corps et un conducteur électrique, notamment un fil électriquement conducteur, est guidé par l'implant sur un côté externe du corps et fait l'objet d'un contact électrique sur le côté externe pour la thérapie ou est guidé par l'implant vers un point de contact sous la peau qui fait l'objet d'un contact électrique de l'extérieur par une ponction pour la thérapie ou est guidé par l'implant par le biais d'un redresseur électrique vers une bobine disposée notamment à pas plus de 3°cm sous la peau et connecté à celle-ci de manière électriquement conductrice, dans lesquels la bobine est en outre connectée à la cathode (16) de manière électriquement conductrice par le biais du redresseur électrique, dans lesquels la cathode (16) est également disposée dans le corps, dans lesquels une conduction de courant est induite par induction dans la bobine de l'extérieur du corps pour la thérapie, par laquelle la cathode (16) et l'anode (12) sont mises sous tension.

11. Produits à action antimicrobienne générés au niveau de l'implant selon une des revendications 1 à 9 destinés à être utilisés selon la revendication 1, dans lesquels le champ électrique est établi entre au moins deux plaques d'un condensateur ou par au moins une bobine électrique ou est établi entre une autre anode et une autre cathode au sein du système électrochimique, dans lesquels le système électrochimique comprend en plus outre l'autre anode et l'autre cathode une source de courant connectée à l'autre anode et l'autre cathode de manière électriquement conductrice ou se compose en plus de l'autre anode et de l'autre cathode ainsi que d'une source de courant connectée à l'autre anode et l'autre cathode de manière électriquement conductrice.

12. Produits à action antimicrobienne générés au niveau de l'implant selon la revendication 11 destinés à être utilisés selon la revendication 1, dans lesquels l'électrolyte dans le système électrochimique comprend un fluide auxiliaire électriquement conducteur ou se compose du fluide corporel et du fluide auxiliaire électriquement conducteur, dans lesquels le fluide auxiliaire électriquement conducteur est à chaque fois un fluide réalisant un contact électrique de l'autre anode et l'autre cathode avec le fluide corporel.

13. Produits à action antimicrobienne générés au niveau de l'implant selon une des revendications précédentes destinés à être utilisés selon la revendication 1, dans lesquels l'implant est disposé au sein d'une région fermée du corps, dans lesquels une tension appliquée au système électrochimique est choisie de sorte que la quantité des autres produits gazeux apparaissant au niveau de l'anode est si faible que ceux-ci se dissolvent directement après leur apparition dans le fluide corporel.

14. Produits à action antimicrobienne générés au niveau de l'implant selon une des revendications précédentes destinés à être utilisés selon la revendication 1, dans lesquels l'infection microbienne est une infection microbienne accompagnée d'une réaction inflammatoire.

15. Produits à action antimicrobienne générés au niveau de l'implant selon une des revendications précédentes destinés à être utilisés selon la revendication 1, dans lesquels l'implant est une endoprothèse en un métal.

**Fig. 1**

**Fig. 2**

**Fig. 3**

**Fig. 4**

**Fig. 5**

**Fig. 6**

**Fig. 7**

**Fig. 8**

Kein bakterielles Wachstum ▨  Mäßiges bakterielles Wachstum ▨

Mittleres bakterielles Wachstum ▨  Starkes bakterielles Wachstum ■

**Fig. 9**

Kein bakterielles Wachstum ▨  Mäßiges bakterielles Wachstum ▨

Mittleres bakterielles Wachstum ▨  Starkes bakterielles Wachstum ■

**Fig. 10**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **AUS KRAFT, A.** Doped Diamond: A Compact Review on a New, Versatile Electrode Material. *International Journal of Electrochemical Science,* 2007, vol. 2, 355-385
- **DJAMEL GHERNAOUT.** Microorganisms' Electrochemical Disinfection Phenomena. *EC Microbiology,* 2017, vol. 9.4, 160-169
- **AUS GARRETT, D. et al.** In vivo biocompatibility of boron doped and nitrogen included conductive-diamond for use in medical implants. *J Biomed Mater Res B Appl Biomater,* 10. Januar 2016, vol. 4 (1), 19-26
- **KROMKA, A. et al.** Semiconducting to metallic-like boron doping of nanocrystalline diamond films and its effect on osteoblastic cells. *Diamond & Related Materials,* 2010, vol. 19, 190-195
- **BOOTH, L. et al.** Synthesis and Characterization of Multilayered Diamond Coatings for Biomedical Implants. *Materials (Basel),* Mai 2011, vol. 4 (5), 857-868
- **ALCAIDE, M. et al.** Boron-Doped Nanocrystalline Diamond Electrodes for Neural Interfaces: In vivo Biocompatibility Evaluation. *Frontiers in Neuroscience,* Marz 2016, vol. 10
- **DIRK MOHN et al.** Electrochemical Disinfection of Dental Implants - a Proof of Concept. *PLOS One,* 14. Januar 2011, 1-6
- **PHILIPP SAHRMANN et al.** Effect of Low Direct Current on Anaerobic Multispecies Biofilm Adhering to a Titanium Implant Surface: Electrochemical Implant Disinfection. *CLINICAL IMPLANT DENTISTRY AND RELATED RESEARCH,* 21. November 2012, vol. 16 (4), 552-556